(19) Europäisches Patentamt
European Patent Office
Office européen des brevets

(11) EP 1 310 561 A1

(12) **EUROPEAN PATENT APPLICATION**

(43) Date of publication:
**14.05.2003 Bulletin 2003/20**

(51) Int Cl.[7]: **C12N 15/85**, A61K 48/00

(21) Application number: **02022942.3**

(22) Date of filing: **11.10.2002**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **09.11.2001 EP 01440378**
**21.11.2001 US 331767 P**

(71) Applicant: **TRANSGENE S.A.**
**67082 Strasbourg Cédex (FR)**

(72) Inventors:
- **Neuville, Pascal**
  **67610 La Wantzenau (FR)**
- **Ribault, Sébastien**
  **13380 Plan De Cuques (FR)**
- **Calenda, Valérie**
  **67000 Strasbourg (FR)**
- **Frauli, Mélanie**
  **67100 Strasbourg (FR)**

(74) Representative: **VOSSIUS & PARTNER**
**Siebertstrasse 4**
**81675 München (DE)**

# (54) Chimeric promoters for controlling expression in skeletal muscle cells

(57) The present invention concerns chimeric constructs comprising a skeletal alpha-actin gene promoter operably linked with at least a skeletal muscle-specific enhancer of a human gene. It also provides an expression cassette comprising such a chimeric construct to control expression of a gene of interest. The invention also relates to a vector, a viral particle, an eukaryotic host cell, a pharmaceutical composition comprising said expression cassette and their use for specific expression in skeletal muscle cells as well as for therapeutic or prophylactic purposes. Finally, the present invention also provides the therapeutic use of an expression cassette, a vector and a viral particle comprising a gene of interest placed under the control of a skeletal alpha-actin gene promoter and a muscle-specific enhancer, especially for treating peripheral ischemia.

EP 1 310 561 A1

## Description

The present invention concerns a chimeric construct comprising at least a skeletal alpha-actin gene promoter operably linked with a skeletal muscle-specific enhancer of a human gene. It also provides an expression cassette comprising such a chimeric construct to control expression of a therapeutic gene. The invention also relates to a vector, a viral particle, an eukaryotic host cell, a pharmaceutical composition comprising said expression cassette and their use for specific expression in skeletal muscle cells as well as for therapeutic or prophylactic purposes. Finally, the present invention also provides the therapeutic use of an expression cassette, a vector and a viral particle comprising a gene of interest placed under the control of a skeletal alpha-actin gene promoter and a muscle-specific enhancer, especially for treating peripheral ischemia. The present invention relates to the field of tissue-specific gene expression and is useful for many applications including the production of recombinant polypeptides in cultured cell lines, the construction of transgenic animal models, the study of gene regulation and the development of muscle targeting technologies. It is of very special interest in relation to gene therapy, especially for treating muscle-affecting diseases, including cardiovascular disorders.

Cardiovascular diseases are currently the leading cause of death in developed countries. In 2001, a report of the American Heart Association has evaluated that 60 millions of patients suffer from cardiovascular disorders in the USA. Among these patients, 4.5 millions are affected by peripheral ischemia, with 800,000 persons encountering critical stage and 200,000 patients being recommended for limb amputation. Moreover, it is hypothesized that, in the next decade, cardiovascular diseases will also concern the Third World because of industrialisation which is related to the apparition of multiple risk factors such as stress, smoking, lipid-rich diets and physical inactivity.

Cardiovascular disorders mainly originate from atherosclerosis which is, as defined by the World Health Organisation, a modification of the arterial wall due to an accumulation of lipids, carbohydrates, blood platelets, fibrous tissues and calcification. The atherosclerotic plaque or atheroma reduces the arterial lumen, thereby leading to a decreased blood flow downstream of the occlusion. Ischemic diseases are caused by a lack of oxygen resulting from insufficient blood delivery in distant tissues. Chronic ischemic manifestations are lower limb ischemia and angina pectoris. Lower limb ischemia is characterised by rest pain and claudication, whereas the symptom of angina is a discomfort in chest, jaw, shoulder, back and arm. In case of atheroma rupture, a clot can totally obstruct downstream vessels, leading to acute ischemic events such as thrombosis of the compromised limb, heart infarction or stroke, requiring emergency treatments to restore blood flow.

The multifactorial origin of atherosclerosis renders its own treatment and the treatment of the resulting ischemic diseases very difficult. In case of emergency, a surgical approach (bypass surgery, angioplasty with or without stenting) is used to restore blood flow, while current treatments of chronic ischemic diseases are based on vasodilating drugs or anticoagulants. As these treatments are far from being satisfactory, gene therapy represents a real hope. In case of cardiac or peripheral ischemia, a natural compensatory healing process is set up to develop a collateral circulation to palliate the obstructed artery and partially restore blood delivery. Gene therapy strategies are designed to enhance this neoangiogenesis phenomenon using pro-angiogenic factors such as Vascular Endothelial Growth Factor (VEGF) and basic Fibroblast Growth Factor (bFGF). These therapeutic genes are able to promote angiogenesis as they stimulate proliferation, migration and differentiation of the endothelial cells constituting the inner layer of blood vessels (Thomas, 1996, J. Biol. Chem. 271, 603-606).

Gene therapy can be defined as the transfer of nucleic acids to somatic cells of an individual. This transfer can be achieved by different types of vectors either *ex vivo* or *in vivo.* In *ex vivo* strategies, target cells or organs are harvested from the patient and modified *in vitro* before reimplantation. Although these techniques allow very efficient gene transfer, they require patient-tailored treatments and are very expensive. There are only few examples of *ex vivo* cardiovascular gene therapies. For instance, an approach for heart failure treatment consisting in adenovirus-transduction of myoblasts followed by their reinfusion into the heart allowed the persistence of gene expression for several weeks (Taylor et al., 1997, Proc. Assoc. Am. Physicians 109, 245-253). In bypass surgery, *ex vivo* transfection of vein grafts with naked DNA has recently been demonstrated to significantly reduce primary graft failure (Mangi and Dzau, 2001, Ann. Med. 33, 153-155). *In vivo* strategies involve a direct administration of gene transfer vectors to the patient. This can be done either systemically or directly into the target tissue, therefore increasing safety and gene transfer efficiency. Gene therapy for ischemic diseases is mainly performed *in vivo.* In case of peripheral ischemia, a direct intramuscular injection in the ischemic region is performed to attract endothelial cells from pre-existing vessels in order to enhance revascularization of the compromised region. Moreover, the development of catheterization allows a controlled and focal injection in case of both peripheral and cardiac ischemia.

Successful gene therapy principally depends on the efficient delivery of the therapeutic gene to the cells of a living organism and the expression of the genetic information. Functional genes can be introduced into cells by a variety of techniques resulting in either transient expression or permanent transformation of the host cells with incorporation of said genes into the host genome. The majority of the gene therapy protocols uses viral or synthetic (non-viral) vectors but naked nucleic acids (i.e. plasmid DNA) can also be used for carrying the genes of interest into target cells (Wolff et al., 1990, Science 247, 1465-1468). A phase I clinical trial, consisting in intramuscular injection of naked plasmid DNA encoding VEGF under the control of the cytomegalovirus (CMV) promoter, has demonstrated an improved distal blood flow in 8 out of 10 patients suffering from nonhealing ischemic ulcers and rest pain. Moreover, in 3 patients recommended for below-knee amputation, successful limb salvage was observed (Baumgartner et al., 1998, Circulation 97, 1114-1123).

Synthetic vectors refer to a special combination of nucleic acids (e.g. plasmid DNA) with one or more transfection-facilitating agent(s), such as lipids (DNA-lipoplex or liposomes) or polymers (DNA-polyplex) which facilitate cellular uptake of the vector. Various lipid- and/or polymer-based vectors are currently available (see for example Rolland, 1998, Critical Reviews in Therapeutic Drug Carrier Systems 15, 143-198 ; Wagner et al., 1990, Proc. Natl. Acad. Sci. USA 87, 3410-3414 and Gottschalk et al., 1996, Gene Ther. 3, 448-457). Although less efficient than viral vectors, synthetic vectors present potential advantages with respect to large-scale production, safety, low immunogenicity and cloning capacity (Ledley, 1995, Human Gene Ther. 6, 1129-1144).

Viruses have developed diverse and highly sophisticated mechanisms to achieve transport across the cellular membrane, to escape from lysosomal degradation, for delivery of their genome to the nucleus and, consequently, have been used in many gene delivery applications. While those derived from retroviruses, adeno-associated viruses (AAV) and adenoviruses have been extensively used (for reviews, see Crystal, 1995, Science 270, 404-410 ; Kovesdi et al., 1997, Curr. Opinion Biotechnol 8, 583-589 ; Miller, 1997, Human Gene Ther. 8, 803-815), other viral vectors such as poxvirus-derived vectors, are emerging as promising candidates for *in vivo* gene transfer.

By way of illustration, a retroviral vector engineered to target extracellular matrix (ECM) proteins exposed in pathophysiological lesions have been recently developed. Upon intraarterial instillation, transduction of neointimal cells was demonstrated in a rat model of balloon angioplasty (Hall et al., 2000, Human Gene Ther. 11, 983-993). AAV vectors have also been used for effective gene transfer into the myocardium (Svenssn et al., 1999, Circulation 99, 201-205).

A substantial number of publications report the successful use of adenoviruses in the treatment of vascular diseases. Intramuscular administration of adenovirus vectors expressing vascular endothelial growth factor (VEGF) stimulated angiogenesis in the sitting of hindlimb ischemia in rats (Mack et al., 1998, J. Vasc. Surg 27, 699-709) and in diabetic mice (Rivard et al., 1999, Am. J. Pathol. 154, 355-363). Therapeutic angiogenesis also offers a great promise for coronary artery diseases. In this respect, both fibroblast growth factor-5 and VEGF were shown to induce collateral vessel development and improvement of myocardial perfusion and function in a porcine model of chronic ischemic myocardium, following adenovirus-mediated gene transfer (Mack et al., 1998, J. Thorac. Cardiovasc Surg. 115, 168-177; Gaordano et al., 1996, Nature Med. 2, 534-539). The ability to repeat adenovirus administrations into skeletal muscle for peripheral ischemia gene therapy has been recently demonstrated (Chen et al., 2000, Gene Ther. 7, 587-595) which may improve the efficacy of gene therapies.

However, the broad host range of the present gene therapy vectors can represent a major limitation for their use. This lack of specificity could lead to a widespread expression of the therapeutic genes which might be harmful to the patient, especially when cytotoxic genes are transferred. For example, it has been envisaged that a widespread expression of pro-angiogenic factors could enhance vascularization of pre-existing tumors (Claffey et al., 1996, Cancer Res. 56, 172-181). Supra-physiological VEGF production may induce the formation of hemangiomas (Lee et al., 2000, Circulation 102, 898-901) or the development of angiogenesis-related pathologies, such as retinopathy (Lashkari et al., 2000, Am. J. Pathol. 156, 1337-1344). Thus, means for restricting gene expression to a targeted category of cells would be useful in gene therapy.

Several investigators have proposed to modify vector specificity by attaching ligands which specifically bind to target cell-surface polypeptides (see for example WO94/10323, US 5,543,328 and US 5,770,442). However, this technology is complex and, to be specific, requires the abrogation of the existing interactions between the vector and its naturally-occuring cellular receptor. Up to now, it has not been possible to totally modify the wild-type tropism of the virus, but an enhancement of target cell transduction was reported. For example, the addition of a polylysine motif in the fiber knob resulted in a 4-fold enhancement of muscle cell transduction *in vitro* and *in vivo* (Bouri et al., 1999, Human Gene Ther. 10, 1633-1640). Physical targeting can also be achieved by pseudotyping of the viral capsid, i.e. by the replacement of the fiber by another adenoviral serotype fiber. In particular, it was demonstrated that a serotype 5 adenovirus carrying a serotype 16 fiber was 3-fold and 10-fold more efficient to transduce endothelial cells and smooth muscle cells, respectively, compared to the wild-type virus (Havenga et al., 2001, J. Virol. 75, 3335-3342).

Another alternative is to restrict gene expression to a desired cell population by using tissue-specific transcriptional regulatory elements. This approach is based on restricting the transgene expression to the targeted cell type, whereby infection is not controled.

Numerous tissue-specific promoters/enhancers have been described in the literature that allow the specific and selective expression of genes in various tissues (for a review, see Maniatis et al., 1987, Science 236, 1237-1245 ; Fickett et al., 2000, Current Opinion in Biotechnology 11,19-24) including muscles. Examples of skeletal muscle-specific promoters/enhancers include those isolated from myosin, myogenin (Edmondson et al., 1992, Mol. Cell. Biol. 12, 3665-3677), desmin (European application EP 0 999 278 ; Mericskay et al., 1999, Current Topics in Pathology Vol 93, pp 7-17 ; Eds Desmoulière and Tuchweber, Springer-Verlag Berlin Heidelberg), troponin, beta-enolase (Feo et al., 1995, Mol. Cell. Biol. 15, 5991-6002), creatine kinase (Sternberg et al., 1988, Mol. Cell. Biol. 8, 2896-2909; Trask et al., 1988, J. Biol. Chem. 263, 17142-17149), and skeletal alpha-actin (Taylor et al., 1988, Genomics 3, 323-336) genes

Actin is the major protein component of the sarcomere, the basic unit of muscle fibers. The 2 kilobase pairs (kbp) immediately upstream of the transcription initiation site (+1) are sufficient to program gene expression in a muscle lineage-restricted fashion (Taylor et al., 1988, Genomics 3, 323-336). Moreover, based on serial deletion studies, a 670 bp fragment extending from positions -432 to +239 relative to the transcriptional initiation site was shown to be sufficient to maintain 55% of the activity of the full-length (2 kbp) promoter in myotubes (Muscat al., 1987, Mol. Cell. Biol. 7, 4089-4099).

As demonstrated by Feo et al. (1995, Mol. Cell. Biol. 15, 5991-6002), transcription of the human beta-enolase gene (ENO-3) is regulated by an intronic muscle-specific enhancer, which was identified between positions +504 to +637 in the first intron. Transfection studies demonstrated the ability of this 138 bp element to increase beta-enolase and Herpes Simplex Virus Thymidine Kinase (HSV TK) promoter activities 25- and 3-fold respectively, and to confer in both cases muscle-specific expression to the reporter gene linked thereto.

However, while the afore mentioned transcriptional elements are properly regulated in a muscle-restricted fashion, they are usually weak and provide expression levels much lower (10 to 200-fold) than those obtained with «strong» promoters/enhancers, such as those of Cytomegalovirus (CMV ; Boshart et al., 1985, Cell 41, 521-530) or other viruses (i.e. Rous Sarcoma Virus ; RSV). However, such viral transcriptional elements are usually non-specific, being active in a wide variety of cell types from many species.

Chimeric constructs combining CMV or muscle-specific enhancers with muscle-specific gene promoters have been described in the literature (Barnhart et al., 1998, Human Gene Ther. 9, 2545-2553), including constructs using the skeletal alpha-actin gene promoter. While the association of the CMV enhancer with the human skeletal alpha-actin (HSA) gene promoter significantly improves expression of the reporter gene, both *in vitro* and *in vivo* in muscle cells (when compared to enhancer-less constructs), strong expression is also observed in non-muscle cells, reaching up to 100% of the expression level obtained with the strong CMV promoter/enhancer used as control (Barnhart et al., 1998, Human Gene Ther. 9, 2545-2553 ; Hagstrom et al., 2000, Blood 95, 2536-2542). Moreover, some chimeric constructs exhibiting a strong enhancement *in vitro,* do not reproduce such an increase *in vivo* when injected in skeletal muscles. As mentioned by Barnhart et al., the critical parameters that define gene expression are complex, depending on the nature, number, orientation and position of the enhancer relative to the promoter sequence as well as the sequence environment.

Expression activities of different muscle-specific promoters linked to the mouse muscle creatine kinase enhancer (positions -1351 to -1050 relative to the native transcriptional initiation site) were studied in the context of retroviral vectors for expressing human FIX in skeletal muscles (Wang et al., 1996, Human Gene Therapy 7, 1743-1756). A construct combining the mouse CK enhancer with the skeletal alpha-actin promoter (positions -689 to +86) allowed FIX production into the culture medium of myotubes, but this specific combination performed poorly compared to other mouse CK enhancer/promoter associations.

Non-specific expression that is targeted to non-muscle cells might be incompatible with human gene therapy, especially when delivery of cytotoxic genes or proangiogenic factors is envisaged. Poor expression levels in targeted cells might also compromise the therapeutic benefit expected in the host organism treated.

Altogether, these studies make clear that it is difficult to design an appropriate promoter/enhancer combination allowing significant levels of gene expression while retaining a strict specificity to muscle cells and, preferably, to skeletal muscle cells

Therefore, there is still a need in the art to design transcriptional elements leading to high level and specific expression of genes in muscle cells, especially in the skeletal muscle cells of the ischemic area, in order to achieve therapeutic levels of protein expression and to avoid the potential side effects inherent to a widespread gene expression.

This technical problem is solved by the provision of the embodiments as defined in the claims.

Accordingly, the present invention provides a chimeric construct for the expression of a gene of interest in a host cell or organism comprising at least (i) a skeletal alpha-actin gene promoter operably linked with at least (ii) a skeletal muscle-specific enhancer of a human gene.

The term « chimeric construct » as used herein refers to a nucleic acid construct comprising at least two sequences of various origins referring to species, genes etc. Within the present invention, the terms « nucleic acid» and «polynucleotide» are used interchangeably and define a polymeric form of any length of nucleotides, either deoxyribonucleotides (DNA) or ribonucleotides (RNA) or analogs thereof. Polynucleotides may be single or double stranded, linear or circular. Polynucleotide sequences can be obtained from existing nucleic acid sources (e.g. genomic, cDNA) but can also be synthetic (e.g. produced by oligonucleotide synthesis). A polynucleotide may also comprise modified nucleotides, such as methylated nucleotides or nucleotide analogs (see US 5,525,711, US 4,711,955 or EPA 302 175 as examples of modifications). If present, modifications to the nucleotide structure may be imparted before or after assembly of the polymer. The sequence of nucleotides may also be interrupted by non-nucleotide elements. A polynucleotide may be further modified after polymerization, such as by conjugation with a labeling component.

The term "gene of interest" refers to any gene, the transcription of which is controlled by said chimeric construct of the invention. In particular, the chimeric construct of the invention shows a propensity to direct gene expression in muscle cells, whereas in non-muscle cells, it is not at all or not very active (reduced activity by a factor of at least 5, preferably at least 10 and, more preferably, at least 100 as compared to the expression activity in muscle cells). In this context, the term "muscle-specific expression" refers to a specifically elevated gene expression of the gene of interest in host cells being muscle cells compared to non-muscle cells whereby these host cells may be cultured cells or cells of a host organism. Thus, the chimeric construct of the present invention results in maximal gene expression in muscle cells and thus provides the possibility of transcriptionally targeting expression of said gene of interest preferentially to muscle cells.

A « host cell or organism » is a cell or organism where expression of the gene of interest is expected. Thus, host cells may be for example a cell line used for testing the properties of the gene of interest, a primary cell for genetic modifications *ex vivo* or a cell within a human or animal organism that will be modified by *in vivo* introduction of a vector carrying the chimeric construct of the invention. In the context of the present invention, the host cell preferably is a muscle cell.

« Muscle » refers to any types of muscles, including skeletal, cardiac and smooth muscles. "Smooth muscles» include visceral and vascular smooth muscles and, especially, arterial smooth muscle cells (SMCs), with a special preference for neointimal and medial SMCs of the aorta, coronary, mammary, femoral and carotid arteries as well as of the saphenous vein. « Skeletal muscle cells » include myoblasts, myotubes, myofibers, myofibrills and satellite cells. «Cardiac muscle cells» include cardiomyocytes and satellite cells. Skeletal muscles are preferred in the context of the present invention.

"Operably linked" refers to a juxtaposition of the elements of the chimeric construct of the invention permitting them to mediate muscle-specific gene expression of a gene of interest placed under the control of said chimeric construct. For instance, an enhancer is operably linked to a promoter if the enhancer enhances transcription of the associated gene, resulting in an enhancement of the expression of the associated gene in the host cell or organism. However, the term "operably linked" as used herein also refers to the juxtaposition of a gene of interest with the sequences controlling its transcription. For instance, a promoter is operably linked to a gene of interest if the promoter allows transcription of the gene in the host cell or organism. There may be additional residues between the promoter and the gene of interest so long as this functional relationship is preserved.

The nucleotide positions referenced in the present application for the cited promoters and enhancers are numbered relative to the presumed transcription initiation site (or cap site representing position +1) of the (native) gene concerned. By way of illustration, the first nucleotide directly upstream from the transcription initiation site is numbered -1 whereas the nucleotide following it is numbered +2. The initiation site of transcription can be determined by standard techniques such as S1 mapping or primer extension (Sambrook et al., 1989, Laboratory Manual, Cold Spring Harbor Laboratory Press, Cold Spring Harbor NY).

The present invention discloses a chimeric construct for the tissue-specific expression of genes of interest using a skeletal alpha-actin gene promoter together with a human muscle-specific enhancer. Preferably, the present invention results in maximal expression in muscle cells or tissues, and especially skeletal muscle cells or tissues, compared to non-muscle cells or tissues as discussed above.

In the natural context, the "skeletal alpha-actin gene promoter" controls the expression of transcripts encoded by the skeletal alpha-actin open reading frame. The promoter used in the chimeric construct of the invention may be any nucleic acid sequence isolated or obtained from the 5' flanking region immediately upstream of the coding sequence of a native skeletal alpha-actin gene and capable of promoting transcription of a gene placed under its control. Within the context of the present invention, such a native promoter sequence can be further modified as it is described further below.

The skeletal alpha-actin gene promoter used in the present invention encompasses at least the elements necessary and/or sufficient to promote gene expression, even at low levels, in muscle cells. Such a promoter may be a so-called "minimal promoter" which includes the *cis*-acting sequences necessary to allow RNA polymerase binding and to initiate transcription at the cap site, such as a TATA box (consensus sequence TATAAAA) or a TATA box-like element (an AT rich sequence having a TATA box function), preferably located 25-35 bp upstream of the cap site. The presence of a TATA box can be determined by sequence analysis whereas the initiation site of transcription can be determined by the precited standard techniques (S1 mapping or primer extension). Preferably, the skeletal alpha-actin gene promoter is usually comprised within a 200 base pairs (bp) fragment, advantageously, within a 100 base pairs (bp) fragment located 5' (upstream) to the transcription initiation site and may extend further in either the 5' or the 3' or both the 5' and the 3' direction.

According to a second and preferred alternative, the chimeric construct of the present invention employs a skeletal alpha-actin gene promoter containing additional cis-acting sequences which allow to substantially increase gene expression directed by a minimal promoter in muscle cells. Such cis-acting sequences can be bound by transcription factors which are either ubiquitous or have a regulatory role (temporal or tissue-specific), especially by muscle-specific transcription factors. Representative examples include, without limitation, CAAT box (consensus GGCCAATCT) bound by the NF-1 factor, GC box (consensus GGGCGG) bound by the SP1 factor, octamer ATTTGCAT bound by the Oct factor, κB (consensus GGGACTTTCC) bound by NFκB, ATF (consensus GTGACGT) bound by the ATF factor Ap2, Sp1, Egr1, YY1, TGT3-3, E box (CANNTG), CarG box ($CC(A/T)_6GG$) and/or MEF-2 (YTAWAAATAR) sequences. These cis-acting sequences may be used alone or in various combinations and may be homologous (isolated from the skeletal alpha-actin gene promoter in use in the present invention) or heterologous (isolated from another promoter region). In this context, it may be advantageous to fuse different portions of several muscle-specific promoters in order to optimize gene expression provided by the skeletal alpha-actin gene promoter.

According to this preferred embodiment, a skeletal alpha-actin gene promoter in use in the chimeric construct of the invention is contained within a 1000 bp fragment, advantageously within a 800 bp fragment, preferably within a 500 bp fragment and, more preferably within a 432 bp fragment naturally located upstream of the transcription initiation site of said skeletal alpha-actin gene.

Furthermore, the skeletal alpha-actin gene promoter in use in the chimeric construct of the present invention may comprise a transcription initiation site functional in the muscle cells, if not present in the adjacent gene of interest. Preferably, the chimeric construct of the present invention employs the naturally-occuring transcription initiation site of the skeletal alpha-actin gene from which the promoter originates.

The skeletal alpha-actin gene promoter sequences useful in accordance with the present invention may be cloned using known recombinant techniques, from naturally occurring vertebrates, preferably humans, and optionally modified as explained in the following.

Accordingly, the isolated DNA fragment in use in the present invention may comprise a number of variations compared to the native sequence of the skeletal alpha-actin gene promoter region of a given organism, provided that these variations do not alter the transcription function conferred by the native sequence. For example, the regions isolated from different species, subspecies or strains of an organism may possess sequence polymorphisms that render those sequences substantially the same as, but not identical to, the native sequences set forth herein. Accordingly, the present invention is intended to encompass all variants displaying the addition, deletion and/or substitution of one or more nucleotide(s) of the native skeletal alpha-actin gene promoter within the confines of appropriate levels of sequence homology. The variant sequences contemplated herein should possess at least about 70% sequence identity, preferably at least 80%, more preferably at least 90% and still more preferably at least 95% identity to the naturally occurring and/or examplified sequences or fragments thereof described herein, with a special preference for an absolute identity (100% identity).

To determine this identity, the variant and native sequences are aligned so as to obtain a maximum match using gaps and inserts. Two sequences are said to be «identical » if the sequence of residues is the same when aligned for maximum correspondence as described below. Optimal alignment of sequences for comparison can be conducted by the local homology algorithm of Smith and Waterman (1981, Adv. Appl. Math. 2, 482-489), by the homology alignment method of Needlemen and Wunsch (1970, J. Mol. Biol. 48, 443-453), by the search for similarity method of Pearson and Lippman (1988, Proc. Natl. Acad. Sci. USA 85, 2444-2448) or the like. Computer implementations of the above algorithms are known as part of the Genetics Computer Group (GCG) Wisconsin genetics Software Package (GAP, BESTFIT, BLASTA, FASTA, TFASTA and FASTDB, Madison, WI). These programs are preferably run using default values for all parameters. A preferred method for determining the best overall match between the variant and the native sequence can be determined using the FASTDB computer program based on the algorihm of Brutlag et al. (1990, Comp. App. Biosci. 6, 237-245).

The chimeric construct of the present invention may employ a skeletal alpha-actin gene promoter from any species, and in particular from human, mouse, rat, hamster, rabbit or pig. The sequence of the skeletal alpha-actin gene promoter of various species is accessible to the man skilled in the art from literature or specialized data banks (such as Genebank under accession numbers M20543 for the human sequence, X67686 for the mouse sequence and V01218 for the rat sequence). The position of the key cis-acting sequences can be determined on the basis of the published data.

According to a preferred embodiment, the chimeric construct of the present invention comprises a skeletal alpha-actin gene promoter obtained from the human gene, and especially a human skeletal alpha-actin gene promoter comprising a nucleotide sequence as shown in SEQ ID NO : 1 from positions 1 to 432 (corresponding to the 432 bp fragment of the human skeletal alpha-actin gene promoter upstream of the transcription initiation site) or a portion thereof having substantially the same transcription-promoting activity as the 432 bp fragment. By way of illustration, an advantageous portion thereof comprises at least a minimal skeletal alpha-actin gene promoter as described above. The human skeletal alpha-actin gene promoter can be isolated as described in Taylor et al. (1988, Genomics 3, 323-336). The other mammalian forms of the skeletal alpha-actin gene promoter can be obtained using conventional molecular biology techniques or by PCR from an appropriate template (e.g a prior art plasmid as described in the precited literature or genomic DNA). For example, fragments of the human gene can be used to probe a genomic library made from other species of mammals under conditions allowing homologous sequences to hybridize. Appropriate hybridization conditions can be determined by reference to standard manuals (e.g. Sambrook et al., 1989, Molecular cloning, Cold Spring Harbor, NY).

The term "skeletal muscle-specific enhancer of a human gene" as used herein refers to a nucleotide sequence to which (a) factor(s) bind(s) directly or indirectly (i.e. through interaction with another cellular factor), thereby enhancing gene expression driven by the skeletal alpha-actin gene promoter present in the chimeric construct of the invention, especially in muscle cells. Such an enhancement can be determined for example by comparing the expression of a reporter gene under the control of the skeletal alpha-actin gene promoter in the presence and in the absence of the skeletal muscle-specific enhancer of a human gene, either *in vitro* (e.g. in cultured muscle cells) or *in vivo* (e.g. in transgenic animals or by direct administration to animal models) and under the same experimental conditions. Examples of such gene expression analysis is provided in the Example section of the present specification, however other methods well known to those skilled in the art are also usable in the context of the invention.

A large number of skeletal muscle-specific enhancers from human sources are well known in the art and available as or within cloned nucleotide sequences (e.g. from depositories such as ATCC or other commercial or individual sources). Accordingly, in a preferred embodiment, such an enhancer is selected from the group of enhancers of the human genes encoding :

a) creatine kinase (Trask et al., 1988, J. Biol. Chem., 263, 17142-17149 ; Genbank accession number AH003460). A preferred creatine kinase enhancer is the sequence as shown in SEQ ID NO : 2 (corresponding to positions approximately -919 to approximately -711 upstream of the transcription initiation site of the human creatine kinase gene) or a portion thereof;
b) beta-enolase, especially the human ENO-3 gene with a special preference for the enhancer fragment comprising the sequence as shown in SEQ ID NO: 3 (corresponding to positions approximately +504 to approximately +637 downstream of the transcription initiation site in the first intron of the human beta-enolase gene ; Feo et al., 1995, Mol. Cell Biol. 15, 5991-6002) or a portion thereof ;
c) myogenin (Genbank accession number X62155);
d) troponin, especially troponin C (Genbank accession number M37984), I (Genbank accession number X90780) or T (Genbank accession number AJ011712).

As discussed hereinafter, the term « portion thereof» encompasses any functional portion of the enhancer sequence defined above, provided that the enhancer activity be substantially preserved (at least 80% of the activity conferred by the native sequence).

As previously mentioned, the human skeletal muscle-specific enhancer is operably linked with the skeletal alpha-actin gene promoter if the enhancer increases gene expression driven by the promoter. An operably linked enhancer can be placed in the chimeric construct upstream or downstream of the promoter within the gene sequence or downstream of said gene sequence. Furthermore, the enhancer can be adjacent, at a close distance or over a distance of up to several kb to the skeletal alpha-actin gene promoter. Advantageously, the human skeletal muscle-specific enhancer is positioned upstream of the skeletal alpha-actin gene promoter, advantageously with a distance separating the promoter and the enhancer by less than 500 bp, preferably less than 200 bp and, more preferably immediately adjacent to the promoter. Moreover, the orientation of the human skeletal muscle-specific enhancer may be sense (5'->3') or antisense (3'->5') relative to the transcriptional direction conferred by the skeletal alpha-actin gene promoter. The optimal location and orientation of each element present in the chimeric construct of the present invention relative to the others can be determined by routine experimentation for any particular chimeric construct. In the context of the present invention, the chimeric construct preferably comprises the enhancer (e.g. the creatine kinase or the beta-enolase enhancer) positioned in antisense orientation relative to the skeletal alpha-actin gene promoter. The beta-enolase enhancer can also be inserted downstream of the skeletal alpha-actin gene promoter (i.e. as an intron or within an intron). Also encompassed are chimeric constructs which contain more than one human skeletal muscle-specific enhancer as defined hereinabove.

The operability of the chimeric construct of the present invention may be easily determined by measuring its capability to drive gene expression (e.g. of a reporter gene encoding for example the bacterial enzyme chloramphenicol acetyltransferase (CAT), β-galactosidase, luciferase or eGFP) in muscle cells, either *in vitro* in appropriate cultured cells or *in vivo* (in transgenic animals or by direct administration to animal models). Gene expression can be determined by standard methods such as flow cytometry, ELISA, immunofluorescence, Western blotting, biological activity measurement and the like.

Moreover, the gene expression activity of the chimeric construct of the invention can be compared to strong promoters/enhancers, such as those of the virus CMV or RSV. More specifically, the chimeric construct of the present invention employing the human beta-enolase enhancer provides expression levels of the reporter gene in cultured myotubes of at least 30%, advantageously at least 40%, preferably at least 70% and more particularly approximately 80% of that obtained with the CMV promoter/enhancer in cultured myotubes under comparable experimental conditions. When using the human skeletal alpha-actin gene promoter linked with the human creatine kinase enhancer, reporter gene expression in myotubes can reach at least 50%, and preferably between 75 to 85% of the expression levels obtained with the reference CMV promoter/enhancer and, at least 100%, and preferably between 500 to 900% of the expression levels obtained with the reference RSV promoter/enhancer. In non-muscle cells, the gene expression driven by the chimeric construct of the present invention is low (less than 5%, advantageously less than 2% and preferably less than 1% of that obtained with the CMV promoter/enhancer) or undetectable.

A chimeric construct of the present invention can be constructed by standard molecular biology techniques well known in the art. The skeletal alpha-actin promoter and the human skeletal muscle-specific enhancer can for example be isolated by cloning techniques from DNA libraries or by amplification methods (PCR) using appropriate probes or primers. Alternatively, they may be produced by chemical synthesis based upon sequence data available in the art. The promoter- and enhancer-bearing fragments can be associated by means of using restriction enzymes and ligases to generate the chimeric construct of the invention.

In the context of the present invention, the promoter or enhancer for use in the chimeric construct or both can be modified by deletion, addition and/or substitution of one or several nucleotide(s), provided that their respective activity as defined above be substantially preserved (at least 80% of the activity of the native sequence). Such modifications can be aimed to remove (i) positive cis-acting sequences which control expression in cell types other than muscle cells, especially skeletal muscle cells, in order to improve muscle-specificity ; or (ii) negative cis-acting sequences (« silencers ») which reduce expression levels. Site-directed mutagenesis can be used to modify the native sequence.

The present invention also provides an expression cassette comprising a gene of interest placed under the control of a chimeric construct according to the invention, allowing its expression in a host cell or organism, preferably in a muscle cell or tissue.

In addition to the definition given futher above, the term " gene of interest " refers to a nucleic acid which can be of any origin and isolated from a genomic DNA, a cDNA, or any DNA encoding a RNA, such as a genomic RNA, an mRNA, an antisense RNA, a ribosomal RNA, a ribozyme or a transfer RNA. The gene of interest can also be an oligonucleotide (i.e. a nucleic acid having a short size of less than 100 bp).

In a preferred embodiment, the gene of interest in use in the present invention, is a therapeutic gene, i.e. encodes a gene product of therapeutic interest. A "gene product of therapeutic interest" is one which has a therapeutic or protective activity when administered appropriately to a patient, especially a patient suffering from a disease or illness condition or who should be protected against a disease or condition. Such a therapeutic or protective activity can be correlated to a beneficial effect on the course of a symptom of said disease or said condition. It is within the reach of the man skilled in the art to select a gene encoding an appropriate gene product of therapeutic interest, depending on the disease or condition to be treated. In a general manner, his choice may be based on the results previously obtained, so that he can reasonably expect, without undue experimentation, i.e. other than practicing the invention as claimed, to obtain such therapeutic properties.

In the context of the invention, the gene of interest can be homologous or heterologous with respect to to the target cell or organism into which it is introduced. Advantageously, it encodes a polypeptide, a ribozyme or an antisense RNA. The term «polypeptide» is to be understood as any translational product of a polynucleotide whatever its size is, and includes polypeptides having as few as 7 amino acid residues (peptides), but more typically proteins. In addition, it may be of any origin (prokaryotes, lower or higher eukaryotes, plant, virus etc). It may be a native polypeptide, a variant, a chimeric polypeptide having no counterpart in nature or fragments thereof. Advantageously, the gene of interest in use in the present invention encodes at least one polypeptide that can compensate for one or more defective or deficient cellular proteins in an animal or a human organism, or that acts through toxic effects to limit or remove harmful cells from the body. A suitable polypeptide may also be immunity conferring and acts as an antigen to provoke a humoral or a cellular response, or both.

Examples of polypeptides encoded by the gene of interest in use in the expression cassette of the present invention include without limitation polypeptides selected from the group consisting of:

- proangiogenic polypeptides such as members of the family of vascular endothelial growth factors (VEGF), transforming growth factor (TGF, and especially TGFa and b), epithelial growth factors (EGF), fibroblast growth factor (FGF and especially FGF a and b), tumor necrosis factors (TNF, especially TNF a and b), CCN (including CTGF polypeptides, Cyr61, Nov, Elm-1, Cop-1 and Wisp-3), scatter factor/hepatocyte growth factor (SH/HGF), angiogenin, angiopoïetin (especially 1 and 2), angiotensin-2;
- cytokines, including interleukins (in particular IL-2, IL-8), colony stimulating factors (GM-CSF, G-CSF, M-CSF), interferons (IFN alpha, beta or gamma), plasminogen activator (tPA) and urokinase (uPA) ;
- polypeptides capable of decreasing or inhibiting a cellular proliferation, including antibodies, toxins, immunotoxins, polypeptides inhibiting the oncogen expression products (e.g. ras, map kinase, tyrosine kinase receptors, growth factors), Fas ligand;

- suicide gene products, such as HSV-1 TK (Caruso et al., 1993, Proc. Natl. Acad. Sci. USA 90, 7024-7028 ; Culver et al., 1992, Science 256, 1550-1552), cytosine deaminase (Erbs et al., 1997, Curr. Genet. 31, 1-6 ; WO93/01281 ; EP 402 108), uracil phosphoribosyl transferase (Anderson et al., 1992, Eur. J. Biochem. 204, 51-56 ; Kern et al., 1990, Gene 88, 149-157), and those described in WO96/16183 and WO99/54481 ;
- polypeptides capable of modulating or regulating the expression of cellular genes;
- antigenic polypeptides (e.g. viral immunogenic polypeptides)
- enzymes, such as urease, renin, thrombin, metalloproteinase, nitric oxide synthases eNOS or iNOS, SOD, catalase, heme oxygenase, the lipoprotein lipase family;
- dystrophin or minidystrophin (Sanes et al., 1986, EMBO J. 5, 3133-3142) ;
- markers (beta-galactosidase, CAT, luciferase, GFP etc.); and
- any polypeptides that can be produced and/or secreted from a muscle cell and are recognized in the art as being useful for the treatment or prevention of a clinical condition, more particularly a condition affecting skeletal muscle cells.

It is within the scope of the present invention that the gene of interest may include addition(s), deletion(s) and/or modification(s) of one or more nucleotide(s) with respect to the native sequence.

A preferred embodiment relates to expression cassettes, wherein the gene of interest encodes CTG-F2 (connective tissue growth factor 2; PCT/US01/21799), VEGF (Genbank accession number AY047581) and VEGF-like, bFGF (basic fibroblast growth factor, Genbank accession number NM002006) and bFGF-like, and angiopoïetin 1 (Genbank accession number XM030821) and 2 (Genbank accession number XM034836) polypeptides, preferably each of human origin.

The expression cassette of the present invention may comprise one or more gene(s) of interest. In this regard, the combination of genes encoding proangiogenic polypeptides (e.g. VEGF and bFGF) may be advantageous in the context of the invention. The different genes of interest may be controlled by the chimeric construct of the invention (polycistronic cassette) or by independent promoters, at least one being the chimeric construct as defined above, that are positioned either in the same or in opposite directions. Furthermore, they may be carried by the same vector or by independent vectors.

According to an advantageous embodiment, the expression cassette of the present invention may be engineered to be inducible in response to a particular inducer. According to this embodiment, the expression cassette of the invention contains a gene of interest encoding a ligand placed under the control of the chimeric construct as defined above, said ligand being capable of activating a ligand-regulated promoter controlling expression of a therapeutic gene. The chimeric construct of the invention drives the expression of the ligand in its inactive form. Interaction with the inducer triggers conformational change of the ligand which becomes active. Binding of the ligand to the ligand-regulated promoter induces activation of this promoter which directs the expression of the therapeutic gene. In this approach, transcriptional targeting provided by the chimeric construct of the invention is combined with a ligand-regulated promoter to ensure not only spatial but also temporal control of therapeutic gene product production. Many ligand-regulated systems are suitable within the context of the present invention, including tetracycline repressor/operator system and the antiprogestin-regulated gene switch (for a review, see for example Harvey and Caskey, 1998, Current Opin. Chem. Biol. 2, 512-518). The two expression cassettes, i.e. that containing respectively the chimeric construct which drives ligand expression and that containing a ligand-regulated promoter which drives therapeutic gene expression, can be introduced into the same vector (i.e. in E1 and E3-deleted adenoviral vector) or into independent vectors.

The expression cassette of the present invention may further comprise additional functional elements such as exon/intron sequences, targeting sequences, transport sequences, secretion signal sequences, nuclear localization signal sequences, IRES, polyA transcription termination sequences, tripartite leader sequences, sequences involved in replication or integration. Said sequences have been reported in the literature and can be readily obtained by those skilled in the art.

In a preferred embodiment, the expression cassette of the invention further comprises one or more exon(s) or (a) portion(s) thereof, preferably, non-coding exon(s), and optionally, one or more intron(s). Such exon and/or intron sequences may be advantageous for stabilizing expression and may for example be obtained from one of the elements contained in the chimeric construct of the invention (the skeletal alpha-actin gene or the human gene from which the skeletal muscle-specific enhancer is obtained) or from any other origin (e.g. eukaryotic, viral, synthetic). The large variety of exon/intron sequences described in the state of the art is suitable in the context of the present invention. They are preferably inserted behind the transcription initiation site and before the gene of interest.

Referring to the preferred embodiment of the chimeric construct that comprises the human skeletal alpha-actin gene promoter, an appropriate exon sequence comprises the portion of the first non-coding exon extending from position +1 to approximately position +239 of the human skeletal alpha-actin gene (i.e. in SEQ ID NO: 1 from positions 433 to 671).

The expression cassette of the present invention may also contain a polyadenylation signal operably linked to the gene(s) of interest. A polyadenylation sequence is operably linked to the gene to be transcribed, when it allows termination of transcription. It is preferably positioned 3' (downstream) of the gene of interest. A suitable polyadenylation signal includes the beta growth hormone (bGHpA), the SV40 and the beta-globin polyadenylation signals.

The present invention also provides a vector comprising the chimeric construct or the expression cassette according to the invention. The skilled person may choose the appropriate vector out of a wide range of vectors. For instance, the vector may be a naked DNA molecule, for instance in the form of a plasmid or a viral vector, eventually complexed or mixed to one or more transfection-facilitating agent(s). The term "plasmid" denotes an extrachromosomal circular DNA capable of autonomous replication in a given cell. The range of suitable plasmids is very large. Preferably, the plasmid is designed for amplification in bacteria and for expression in a eukaryotic target cell. Such plasmids can be purchased from a variety of manufacturers. Suitable plasmids include but are not limited to those derived from pBR322 (Gibco BRL), pUC (Gibco BRL), pBluescript (Stratagene), pREP4, pCEP4 (Invitrogene), pCI (Promega) and p Poly (Lathe et al., 1987, Gene 57, 193-201). It can also be engineered by standard molecular biology techniques (Sambrook et al., 1989, Laboratory Manual, Cold Spring Harbor Laboratory Press, Cold Spring Harbor NY). It may also comprise a selection gene in order to select or to identify the transfected cells (e.g. by complementation of a cell auxotrophy or by antibiotic resistance), stabilizing elements (e.g. *cer* sequence; Summers and Sherrat, 1984, Cell 36, 1097-1103) or integrative elements (e.g. LTR viral sequences and transposons).

A preferred embodiment of the vectors of the invention relates to viral vectors derived from a virus selected from the group consisting of herpes viruses, cytomegaloviruses, foamy viruses, lentiviruses, Semliki forrest virus, AAV (adeno-associated virus), poxviruses, retroviruses and adenoviruses. Such viral vectors are well known in the art. « Derived » means genetically engineered from the native viral genome by introducing one or more modifications, such as deletion(s), addition(s) and/or substitution(s) of one or several nucleotide(s) present in a coding or a non-coding portion of the viral genome.

A viral vector which is particularly appropriate for the present invention is an adenoviral vector. The adenoviral genome consists of a linear double-stranded DNA molecule of approximately 36kb carrying more than about thirty genes necessary to complete the viral cycle. The early genes are divided into 4 regions (E1 to E4) that are essential for viral replication (Pettersson and Roberts, 1986, In Cancer Cells (Vol 4): DNA Tumor Viruses, Botchan and Glodzicker Sharp Eds pp 37-47, Cold Spring Harbor Laboratory, Cold Spring Harbor, N.Y. ; Halbert et al., 1985, J. Virol. 56, 250-257) with the exception of the E3 region, which is believed dispensable for viral replication based on the observation that naturally-occuring mutants or hybrid viruses deleted within the E3 region still replicate like wild-type viruses in cultured cells (Kelly and Lewis, 1973, J. Virol. 12, 643-652). The E1 gene products encode proteins responsible for the regulation of transcription of the viral genome. The E2 gene products are required for initiation and chain elongation in viral DNA synthesis. The proteins encoded by the E3 prevent cytolysis by cytotoxic T cells and tumor necrosis factor (Wold and Gooding, 1991, Virology 184, 1-8). The proteins encoded by the E4 region are involved in DNA replication, late gene expression and splicing and host cell shut off (Halbert et al., 1985, J. Virol. 56, 250-257). The late genes (L1 to L5) encode in their majority the structural proteins constituting the viral capsid. They overlap at least in part with the early transcription units and are transcribed from a unique promoter (MLP for Major Late Promoter). In addition, the adenoviral genome carries at both extremities *cis*-acting 5' and 3' ITRs (Inverted Terminal Repeat) and packaging sequences essential for DNA replication. The ITRs harbor origins of DNA replication whereas the encapsidation region is required for the packaging of adenoviral DNA into infectious particles.

In one embodiment, the adenoviral vector of the present invention is engineered to be conditionally replicative (CRAd vectors) in order to replicate selectively in specific cells (e.g. proliferative cells) as described in Heise and Kirn (2000, J. Clin. Invest. 105, 847-851).

According to another and preferred embodiment, the adenoviral vector of the invention is replication-defective, at least for the E1 function by total or partial deletion and/or mutation of one or more genes constituting the E1 region. Advantageously, the E1 deletion covers nucleotides (nt) 458 to 3328 or 458 to 3510 by reference to the sequence of the human adenovirus type 5 disclosed in the Genebank data base under the accession number M 73260.

Furthermore, the adenoviral backbone of the vector may comprise additional modifications (deletions, insertions or mutations in one or more viral genes). An example of an E2 modification is illustrated by the thermosensible mutation localized on the DBP (DNA Binding Protein) encoding gene (Ensinger et al., 1972, J. Virol. 10, 328-339). The adenoviral sequence may also be deleted of all or part of the E4 region. A partial deletion retaining the ORFs 3 and 4 or ORFs 3 and 6/7 may be advantageous (see for example European application EP 974 668 ; Christ et al., 2000, Human Gene Ther. 11, 415-427 ; Lusky et al., 1999, J. Virol. 73, 8308-8319). Additional deletions within the non-essential E3 region may increase the cloning capacity (for a review see for example Yeh et al. FASEB Journal 11 (1997) 615-623), but it may be advantageous to retain all or part of the E3 sequences coding for the polypeptides (e.g. gpl9k) allowing to escape the host immune system (Gooding et al., 1990, Critical Review of Immunology 10, 53-71) or inflammatory reactions (EP00440267.3). Second generation vectors retaining the ITRs and packaging sequences and containing substantial genetic modifications aimed to abolish the residual synthesis of the viral antigens may also be envisaged, in order to improve long-term expression of the expressed gene in the transduced cells (WO94/28152 ; Lusky et al., 1998, J. Virol 72, 2022-2032).

The expression cassette of the present invention can be inserted in any location of the adenoviral genome, with the exception of the *cis*-acting sequences. Preferably, it is inserted in replacement of a deleted region (E1, E3 and/or E4), with a special preference for the deleted E1 region. In addition, the expression cassette may be positioned in sense or antisense orientation relative to the transcriptional direction of the region in question.

Adenoviruses adaptable for use in accordance with the present invention, can be derived from any human or animal source, in particular canine (e.g. CAV-1 or CAV-2 ; Genbank ref CAV1GENOM and CAV77082 respectively), avian (Genbank ref AAVEDSDNA), bovine (such as BAV3 ; Seshidhar Reddy et al., 1998, J. Virol. 72, 1394-1402), murine (Genbank ref ADRMUSMAV1), ovine, feline, porcine or simian adenovirus or alternatively from a hybrid thereof. Any serotype can be employed. However, the human adenoviruses of the C sub-group are preferred and especially adenoviruses 2 (Ad2) and 5 (Ad5). Generally speaking, the cited viruses are available in collections such as ATCC and have been the subject of numerous publications describing their sequence, organization and biology, allowing the artisan to apply them.

In addition, adenoviral particles or empty adenoviral capsids can also be used to transfer nucleic acids (e.g. a plasmidic vector) by a virus-mediated cointernalization process as described in US 5,928,944. This process can be accomplished in the presence of (a) cationic agent(s) such as polycarbenes or lipid vesicles comprising one or more lipid layers.

A retroviral vector is also suitable. Retroviruses are a class of integrative viruses which replicate using a virus-encoded reverse transcriptase, to replicate the viral RNA genome into double stranded DNA which is integrated into chromosomal DNA of the infected cells. The numerous vectors described in the literature may be used within the framework of the present invention and especially those derived from murine leukemia viruses, especially Moloney (Gilboa et al., 1988, Adv. Exp.Med. Biol. 241, 29) or Friend's FB29 strains (WO95/01447). Generally, a retroviral vector is deleted of all or part of the viral genes gag, pol and env and retains 5'and 3' LTRs and an encapsidation sequence. These elements may be modified to increase expression level or stability of the retroviral vector. Such modifications include the replacement of the retroviral encapsidation sequence by one of a retrotransposon such as VL30 (US 5,747,323). The expression cassette of the invention is inserted downstream of the encapsidation sequence, preferably in opposite direction relative to the retroviral genome.

Poxviruses are a group of complex enveloped viruses that distinguish from the above-mentioned viruses by their large DNA genome and their cytoplasmic site of replication. The genome of several members of poxviridae has been mapped and sequenced. It is a double-stranded DNA of approximately 200 kb coding for about 200 proteins of which approximately 100 are involved in virus assembly. In the context of the present invention, a poxviral vector may be obtained from any member of the poxviridae, in particular canarypox, fowlpox and vaccinia virus, the latter being preferred. Suitable vaccinia viruses include without limitation the Copenhagen strain (Goebel et al., 1990, Virol. 179, 247-266 and 517-563 ; Johnson et al., 1993, Virol. 196, 381-401), the Wyeth strain and the modified Ankara (MVA) strain (Antoine et al., 1998, Virol. 244, 365-396). The general conditions for constructing a vaccinia virus comprising an expression cassette according to the present invention are well known in the art (see for example EP 83 286 and EP 206 920 for Copenhagen vaccinia viruses and Mayr et al., 1975, Infection 3, 6-14 and Sutter and Moss, 1992, Proc. Natl. Acad. Sci. USA 89, 10847-10851 for MVA viruses).

The expression cassette of the present invention is preferably inserted within the poxviral genome in a non-essential locus, such as non-coding intergenic regions or any gene for which inactivation or deletion does not significantly impair viral growth and replication. Thymidine kinase gene is particularly appropriate for insertion in Copenhagen vaccinia viruses (Hruby et al., 1983, Proc. Natl. Acad. Sci USA 80, 3411-3415 ; Weir et al., 1983, J. Virol. 46, 530-537). As far as MVA is concerned, insertion of the expression cassette can be performed in any of the excisions I to VII, and preferably in excision II or III (Meyer et al., 1991, J. Gen. Virol. 72, 1031-1038 ; Sutter et al., 1994, Vaccine 12, 1032-1040) or in D4R locus. For fowlpox virus, although insertion within thymidine kinase gene may be considered, the expression cassette is preferably introduced into a non-coding intergenic region (see for example EP 314 569 and US 5,180,675). One may also envisage insertion in an essential viral locus provided that the defective function be supplied *in trans*, via a helper virus or by expression in the producer cell line.

According to an advantageous alternative, a vector of the present invention may be complexed to one or more transfection-facilitating agent(s), such as lipids and/or polymers (synthetic vector). Preferred lipids are cationic lipids which have a high affinity for nucleic acids and which interact with cell membranes (Felgner et al., 1989, Nature 337, 387-388). As a result, they are capable of complexing the nucleic acid, thus generating a compact particle capable of entering the cells. Suitable lipids include without limitation DOTMA (Felgner et al., 1987, Proc. Natl. Acad. Sci. USA 84, 7413-7417), DOGS or Transfectam™ (Behr et al., 1989, Proc. Natl. Acad. Sci. USA 86, 6982-6986), DMRIE or DORIE (Felgner et al., 1993, Methods 5, 67-75), DC-CHOL (Gao and Huang, 1991, BBRC 179, 280-285), DOTAP™ (McLachlan et al., 1995, Gene Therapy 2, 674-622), Lipofectamine™ and glycerolipid compounds (see EP901463 and WO98/37916).

Suitable polymers are preferably cationic, such as polyamidoamine (Haensler and Szoka, 1993, Bioconjugate Chem. 4, 372-379), dendritic polymer (WO 95/24221), polyethylene imine or polypropylene imine (WO96/02655), polylysine (US 5 595 897 or FR 2 719 316), chitosan (US 5,744,166) or DEAE dextran (Lopata et al., 1984, Nucleic Acid Res. 12, 5707-5717).

In a further embodiment, the present invention relates to a method for the preparation of viral particles allowing muscle-specific expression of a gene of interest in a host cell or organism, said method comprising the steps of:

a) introducing the viral vector of the invention in a permissive cell line ;
b) culturing the permissive cell line obtained in step a) for an appropriate period of time and under suitable conditions to allow the production of viral particles containing said viral vector ;
c) recovering said viral particles from the cell culture ; and
d) optionally, purifying the recovered viral particles.

In a preferred embodiment, the permissive cell line is a complementation cell line which provides *in trans* all gene products necessary to produce infectious virions.

The present invention also provides viral particles comprising a vector according to the invention, preferably a viral vector, or obtainable by the method for the preparation of such viral particles.

Adenoviral particles may be prepared and propagated according to any conventional technique in the field of the art (e.g. as described in Graham and Prevect, 1991, Methods in Molecular Biology, Vol 7, Gene Transfer and Expression Protocols; Ed E. J. Murray, The Human Press Inc, Clinton, NJ or in WO96/17070) using a complementation cell line or a helper virus, which supplies *in trans* the viral genes for which the adenoviral vector of the invention is defective. The cell lines 293 (Graham et al., 1977, J. Gen. Virol. 36, 59-72) and PERC6 (Fallaux et al., 1998, Human Gene Therapy 9, 1909-1917) are commonly used to complement the E1 function. Other cell lines have been engineered to complement doubly defective vectors (Yeh et al., 1996, J. Virol. 70, 559-565 ; Krougliak and Graham, 1995, Human Gene Ther. 6, 1575-1586 ; Wang et al., 1995, Gene Ther. 2, 775-783 ; Lusky et al., 1998, J. Virol. 72, 2022-2033 ; EP919627 and WO97/04119). The adenoviral particles can be recovered from the culture supernatant but also from the cells after lysis and optionally further purified according to standard techniques (e.g. chromatography, ultracentrifugation, as described in WO96/27677, WO98/00524 WO98/26048 and WO00/50573).

Retroviral particles are prepared in the presence of a helper virus or in an appropriate complementation (packaging) cell line which contains integrated into its genome the retroviral genes for which the retroviral vector is defective (e.g. gag/pol and env). Such cell lines are described in the prior art (Miller and Rosman, 1989, BioTechniques 7, 980 ; Danos and Mulligan, 1988, Proc. Natl. Acad. Sci. USA 85, 6460 ; Markowitz et al., 1988, Virol. 167, 400). The product of the env gene is responsible for the binding of the viral particle to the viral receptors present on the surface of the target cell and, therefore determines the host range of the retroviral particle. In the context of the invention, it is advantageous to use a packaging cell line, such as the PA317 cells (ATCC CRL 9078) or 293E16 (WO97/35996) containing an amphotropic envelope protein, to allow infection of human and other species' target cells. The retroviral particles are preferably recovered from the culture supernatant and may optionally be further purified according to standard techniques (e.g. chromatography, ultracentrifugation).

Poxviral particles are prepared as described in numerous documents accessible to the artisan skilled in the art (Piccini et al., 1987, Methods of Enzymology 153, 545-563 ; US 4,769,330 ; US 4,772,848 ; US 4,603,112 ; US 5,100,587 and US 5,179,993). The major techniques that have been developed utilize homologous recombination between a donor plasmid containing the expression cassette of the invention flanked on both sides by pox DNA sequences (encompassing the desired insertion site) and the wild type poxviral genome. Generally, the donor plasmid is constructed, amplified by growth in *E. coli* and isolated by conventional procedures. Then, it is introduced into a suitable cell culture (e.g. chicken embryo fibroblasts) together with a poxvirus genome, to produce by homologous recombination the poxviral particles of the invention. They can be recovered from the culture supernatant or from the cultured cells after a lysis step (chemical, freezing/thawing, osmotic shock, mechanic shock, sonication and the like) and can be, if necessary, isolated from wild type contamination by consecutive rounds of plaque purification and then purified using the techniques of the art (chromatographic methods, ultracentrifugation on cesium chloride or sucrose gradient).

The present invention also encompasses vectors or particles that have been modified to allow preferential targeting of a particular target cell. A characteristic feature of targeted vectors/particles of the invention (of both viral and non-viral origins, such as polymer- and lipid-complexed vectors) is the presence at their surface of a targeting moiety capable of recognizing and binding to a cellular and surface-exposed component or to the extracellular matrix (ECM) such as collagen (Hall et al., 2000, Human Gene Therapy 11, 983-993). Such targeting moieties include without limitation chemical conjugates, lipids, glycolipids, hormones, sugars, polymers (e.g. PEG, polylysine, polyethylenimine and the like), peptides, polypeptides (for example JTS1 as described in WO 94/40958), oligonucleotides, vitamins, antigens, lectins, antibodies and fragments thereof. They are preferably capable of recognizing and binding to cell-specific markers, tissue-specific markers, cellular receptors, viral antigens, antigenic epitopes or tumor-associated markers.

Cell type-specific targeting may be achieved with vectors derived from viruses having a broad host range by the modification of viral surface proteins. For example, the specificity of infection of adenoviruses is determined by the attachment to cellular receptors present at the surface of permissive cells. In this regard, the fiber and penton present at the surface of the adenoviral capsid play a critical role in cellular attachment (Defer et al., 1990, J. Virol. 64, 3661-3673). Thus, cell targeting of adenoviruses can be carried out by genetic modification of the viral gene encoding fiber and/or penton, to generate modified fiber and/or penton capable of specific interaction with unique cell surface receptors. Examples of such modifications are described in literature (for example in Wickam et al., 1997, J. Virol. 71, 8221-8229 ; Arnberg et al., 1997, Virol. 227, 239-244 ; Michael et al., 1995, Gene Therapy 2, 660-668 ; WO94/10323, WO01/16344 and WO01/38361). To illustrate, inserting a sequence coding for EGF within the sequence encoding the adenoviral fiber will allow to target EGF receptor expressing cells

Other methods for cell specific targeting have been achieved by the conjugation of antibodies or antibody fragments to the retroviral envelope protein (Michael et al., 1993, J. Biol. Chem 268, 6866-6869 ; Roux et al., 1989, Proc. Natl. Acad Sci. USA 86, 9079-9083 ; Miller and Vile, 1995, FASEB J. 9, 190-199 and WO93/09221) and of polypeptides having a nucleic acid binding domain and a targeting moiety (WO95/28494).

The present invention also provides a eukaryotic host cell comprising the chimeric construct, the expression cassette or the vector according to the invention or infected by the viral particle according to the invention. In the context of the present invention, the term " eukaryotic host cell" designates any cell comprising one or several transcriptional factors capable of interacting with the cis-acting sequences present in the skeletal alpha-actin gene promoter and/or the human skeletal muscle-specific enhancer in use in the present invention. Such cells may be of a unique type of cells or a group of different types of cells and encompass cultured cell lines, primary cells and proliferative cells from mammalian origin, with a special preference for human origin. Preferred eukaryotic host cells include fibroblasts and muscle cells (as defined above) and, especially, skeletal muscle cells.

Moreover, according to a specific embodiment, the eukaryotic host cell is further encapsulated. Cell encapsulation technology has been previously described (Tresco et al., 1992, ASAIO J. 38, 17-23 ; Aebischer et al., 1996, Human Gene Ther. 7, 851-860). According to said specific embodiment, transfected or infected host cells are encapsulated with compounds which form a microporous membrane and said encapsulated cells can further be implanted in vivo. Capsules containing the cells of interest may be prepared employing a hollow microporous membrane from poly-ether sulfone (PES) (Akzo Nobel Faser AG, Wuppertal, Germany ; Deglon et al. 1996, Human Gene Ther. 7, 2135-2146). This membrane has a molecular weight cutoff greater than 1Mda which permits the free passage of proteins and nutrients between the capsule interior and exterior, while preventing the contact of transplanted cells with host cells.

The present invention also provides a pharmaceutical composition comprising the chimeric construct, the expression cassette, the vector, the viral particle or the eukaryotic host cell according to the present invention and, optionally, a pharmaceutically acceptable carrier. In a special case, the composition may comprise two or more expression cassettes, vectors, viral particles or eukaryotic host cells, which may differ by the nature of (i) the expression cassette (i.e. ligand-regulated system), (ii) the human skeletal muscle-specific enhancer (iii) the skeletal alpha-actin gene promoter (iv) the gene of interest and/or (v) the vector backbone.

The composition according to the invention may be manufactured in a conventional manner for a variety of modes of administration including systemic, topical and localized administration. For systemic administration, injection is preferred, e.g. subcutaneous, intradermal, intramuscular, intravenous, intraperitoneal, intrathecal, intracardiac (such as transendocardial and pericardial), intratumoral, intravaginal, intrapulmonary, intranasal, intratracheal, intravascular, intraarterial, intracoronary or intracerebroventricular. Intramuscular constitutes the preferred mode of administration. The administration may take place in a single dose or a dose repeated one or several times after a certain time interval. The appropriate administration route and dosage may vary in accordance with various parameters, as for example, the condition or disease to be treated, the stage to which it has progressed, the need for prevention or therapy and the therapeutic gene to be transferred. As an indication, a composition based on viral particles may be formulated in the form of doses of between $10^4$ and $10^{14}$ iu (infectious units), advantageously between $10^5$ and $10^{13}$ iu and preferably between $10^6$ and $10^{12}$ iu. The titer may be determined by conventional techniques. The doses of DNA vector are preferably comprised between 0.01 and 10 mg/kg, more especially between 0.1 and 2 mg/kg. The composition of the invention can be in various forms, e.g. in solid (e.g. powder, lyophilized form), liquid (e.g. aqueous).

In a preferred embodiment, the composition comprises a pharmaceutically acceptable carrier, allowing its use in a method for the therapeutic treatment of humans or animals. In this particular case, the carrier is preferably a pharmaceutically suitable injectable carrier or diluent which is non-toxic to a human or animal organism at the dosage and concentration employed (for examples, see Remington's Pharmaceutical Sciences, 16th ed. 1980, Mack Publishing Co). It is preferably isotonic, hypotonic or weakly hypertonic and has a relatively low ionic strength, such as provided by a sucrose solution. Furthermore, it may contain any relevant solvents, aqueous or partly aqueous liquid carriers comprising sterile, pyrogen-free water, dispersion media, coatings, and equivalents, or diluents (e.g. Tris-HCl, acetate, phosphate), emulsifiers, solubilizers or adjuvants. The pH of the pharmaceutical preparation is suitably adjusted and buffered in order to be appropriate for use in humans or animals. Representative examples of carriers or diluents for an injectable composition include water, isotonic saline solutions which are preferably buffered at a physiological pH (such as phosphate buffered saline, Tris buffered saline, mannitol, dextrose, glycerol containing or not polypeptides or proteins such as human serum albumin). For example, such a composition may comprise 1M saccharose, 150 mM NaCl, 1mM $MgCl_2$, 54 mg/l Tween 80, 10 mM Tris pH 8.5.

In addition, the composition according to the present invention may include one or more stabilizing substance (s), such as lipids (e.g. cationic lipids, liposomes, lipids as described in WO98/44143), nuclease inhibitors, hydrogel, hyaluronidase (WO98/53853), collagenase, polymers, chelating agents (EP890362), in order to preserve its degradation within the animal/human body and/or improve transfection/infection of the vector into the host cell. Such substances may be used alone or in combination (e.g. cationic and neutral lipids). It may also comprise substances susceptible to facilitate gene transfer in arterial cells, such as a gel complex of poly-lysine and lactose (Midoux et al., 1993, Nucleic Acid Res. 21, 871-878) or poloxamer 407 (Pastore, 1994, Circulation 90, I-517). It has also be shown that adenovirus proteins are capable of destabilizing endosomes and enhancing the uptake of DNA into cells. The mixture of adenoviruses to solutions containing a lipid-complexed DNA vector or the binding of DNA to polylysine covalently attached to adenoviruses using protein cross-linking agents may substantially improve the uptake and expression of the recombinant gene (Curiel et al., 1992, Am. J. Respir. Cell. Mol. Biol. 6, 247-252).

The composition of the present invention is particularly intended for the preventive or curative treatment of disorders, conditions or diseases associated with muscles, blood vessels (preferably arteries) and/or the cardiovascular system, including without limitation ischemic diseases (peripheral, lower limb, cardiac ischemia and angina pectoris), artherosclerosis, lhypertension, atherogenesis, intimal hyperplasia, (re)restenosis following angioplasty or stent placement, neoplastic diseases (e.g. tumors and tumor metastasis), benign tumors, connective tissue disorders (e.g. rheumatoid arthritis), ocular angiogenic diseases (e.g. diabetic retinopathy, macular degeneration, corneal graft rejection, neovascular glaucoma), cardiovascular diseases, cerebral vascular diseases, diabetes-associated diseases, immune disorders (e.g. chronic inflammation or autoimmunity) and genetic diseases (muscular dystrophies such as Backer and Duchenne, multiple sclerosis). A preferred application is the prevention or treatment of ischemic diseases (acute or chronic). Balloon angioplasty is a major treatment which involves the inflation of a balloon in an occluded blood vessel in order to open the blocked blood vessel. Stent placement is also used to restore blood flow. Unfortunately, these methods of treatment frequently result in injury of the endothelial cells lining the inner wall of blood vessels. Smooth muscle cells often infiltrate into the reopened blood vessels causing a secondary obstruction (a process called restenosis). Virus-mediated gene therapy may be applicable in this case to deliver to the lesion created by the balloon angioplasty or the stenting procedure, a therapeutic gene encoding a product inhibiting SMC proliferation.

The present invention also provides the use of the chimeric construct, the expression cassette, the vector, the viral particle or the eukaryotic host cell according to the invention, for the preparation of a drug for the treatment or the prevention of a disease in a human or animal organism by gene therapy.

Within the scope of the present invention, "gene therapy" has to be understood as a method for introducing any expressible sequence into a cell. Thus, it also includes immunotherapy that relates to the introduction of a potentially antigenic epitope into a cell to induce an immune response which can be cellular or humoral or both.

In a preferred embodiment, such a use is for the treatment or the prevention of a cardiovascular disease, especially peripheral ischemia. For this purpose, the expression cassette, the vector or the viral particle of the present invention may be delivered *in vivo* to the human or animal organism by specific delivery means adapted to this pathology, such as catheters, stents and the like. For example, a balloon catheter or a stent coated with the expression cassette, vector or viral particle of the invention may be employed (as described in Riessen et al., 1993, Hum Gene Ther. 4, 749-758 ; Feldman and Steg, 1996, Medecine/Science 12, 47-55). The catheters suitable for use in the context of the present invention are available from commercial suppliers, such as Advanced Cardiovascular Systems (ACS), Boston Scientific, IVT, Target Therapeutics or Cordis or described in FR 00 08751. By way of illustration, a catheter can be conveniently introduced into a femoral artery and threaded retrograde through the iliac artery and abdominal aorta and into a coronary artery. Detailed descriptions of these techniques can be found in the art (e.g. Rutherford, Vascular Surgery, 3rd edition (Saunders Co 1989). It is also possible to deliver the expression cassette, the vector or viral particle of the present invention directly to the arteries following surgical operation. Another alternative is to introduce along the affected artery a grid frame impregnated with the therapeutic agent (Feldman et al., 1995, J. Clin. Invest. 95, 2662-2671) or to operate via intramuscular injection.

Alternatively, one may employ eukaryotic host cells that have been engineered *ex vivo* to contain the expression cassette, the vector or the viral particle according to the invention. Methods for introducing such elements into an eukaryotic cell are well known to those skilled in the art and include microinjection of minute amounts of DNA into the nucleus of a cell (Capechi et al., 1980, Cell 22, 479-488), transfection with $CaPO_4$ (Chen and Okayama, 1987, Mol. Cell Biol. 7, 2745-2752), electroporation (Chu et al., 1987, Nucleic Acid Res. 15, 1311-1326), lipofection/liposome fusion (Felgner et al., 1987, Proc. Natl. Acad. Sci. USA 84, 7413-7417) and particle bombardement (Yang et al., 1990, Proc. Natl. Acad. Sci. USA 87, 9568-9572). The graft of engineered or encapsulated muscle cells is also possible in the context of the present invention (Lynch et al, 1992, Proc. Natl. Acad. Sci. USA 89, 1138-1142).

The present invention also relates to a method for the treatment of a human or animal organism, comprising administering to said organism a therapeutically effective amount of the expression cassette, the vector, the viral particle or the eukaryotic cell according to the invention.

A « therapeutically effective amount » is a dose sufficient for the alleviation of one or more symptoms normally associated with the disease or condition desired to be treated. When prophylactic use is concerned, this term means a dose sufficient to prevent or to delay the establishment of a disease or condition.

The method of the present invention can be used for preventive purposes and for therapeutic applications relative to the diseases or conditions listed above. The present method is particularly useful to prevent the establishment of ischemia or to reverse ischemia after its onset, using an approach similar to that described herein. It is to be understood that the present method can be carried out by any of a variety of approaches. Advantageously, the expression cassette, the vector or the pharmaceutical composition of the invention can be administered directly *in vivo* by any conventional and physiologically acceptable administration route, for example by intramuscular injection or by means of an appropriate catheter into the vascular system, etc. Alternatively, the *ex vivo* approach may also be adopted which consists of introducing the expression cassette, the vector or the viral particle according to the invention into cells, growing the transfected/infected cells *in vitro* and then reintroducing them into the patient to be treated, eventually after encapsulation in appropriate membrane.

Prevention or treatment of a disease or a condition can be carried out using the present method alone or, if desired, in conjunction with presently available methods (e.g. radiation, chemotherapy and surgery such as angioplasty). In a preferred embodiment, the method acording the invention involves administration into a fluid vessel, such as for example a blood vessel or a lymph vessel, and can be advantageously improved by combining injection in an afferent and/or efferent fluid vessel with increase of permeability of said vessel. In a particular preferred embodiment, said increase is obtained by increasing hydrostatic pressure (i.e. by obstructing outflow and/or inflow), osmotic pressure (i.e. with hypertonic solution) and/or by introducing a biologically active olecule (i.e. histamine into the administered composition; WO98/58542). In a further embodiment, the patient can also be treated with substance facilitating muscle degeneration in order to improve efficacy of the treatment. Furthermore, in order to improve the transfection rate, the patient may undergo a macrophage depletion treatment prior to administration of the composition of the invention. Such a technique is described in literature (for example in Van Rooijen et al., 1997, TibTech, 15, 178-184).

The present invention also provides the use of the chimeric construct, the expression cassette, the vector or the viral particle according to the invention, for specific expression of a gene of interest in skeletal muscle cells.

The present invention also provides a non-human transgenic animal, especially a transgenic mouse, comprising integrated into its genome the expression cassette or the vector according to the invention. Such an animal can be generated by conventional transgenesis methods and can be used as a model to study the potential effect or activity of the therapeutic gene or the regulation of the skeletal alpha-actin gene promoter and/or the human skeletal muscle-specific enhancer present in the expression cassette of the invention.

The present invention also relates to the use of an expression cassette, a vector, a viral particle comprising a gene of interest placed under the control of at least (i) a skeletal alpha-actin gene promoter and (ii) a muscle-specific enhancer, for the preparation of a drug for the treatment or the prevention of a cardiovascular disease in a human or animal organism by gene therapy. A large number of muscle-specific enhancers from a variety of different sources are well known in the art and available as or within cloned polynucleotide sequences (from e.g. depositories such as ATCC or other commercial and individual sources). Apart from those as defined above, suitable muscle-specific enhancers include those operable in smooth muscle, skeletal muscle and cardiac muscle cells. Representative examples include those isolated or derived from the genes encoding :

- a mammalian creatine kinase gene, (i.e. from the mouse gene; Janes et al., 1988, Mol. Cell. Biol. 8, 62-70), whith a special preference for the human creatine kinase enhancer as defined above ;
- a mammalian beta-enolase gene, whith a special preference for the human beta-enolase enhancer as defined above ;
- a mammalian alpha-actin (Shimizu et al., 1995, J. Biol. Chem. 270, 7631-7643), especially the cardiac, the skeletal or the smooth muscle alpha-actin (Genbank accession number D00618);
- a mammalian troponin, especially the troponin C (Genbank accession number M37984), I (Genbank accession number X90780) or T (Genbank accession number AJ011712);
- a mammalian myosin. Several myosin enhancers have been identified to date from both myosin light chain and myosin heavy chain genes (for example Donoghue et al., 1988, Genes and Development 2, 1779-1790). Preferred is a myosin heavy chain enhancer, more preferred one of rabbit, with a special preference for the enhancer located between positions approximately -1332 and approximately -1225 upstream of the transcription initiation site of the rabbit myosin heavy chain encoding gene (Kallmeier et al., 1995, J. Biol. Chem. 270, 30949-30957) ;

- a mammalian APEG-1 (Aortic preferentially expressed gene-1 ; Hsieh et al., 1999, J. Biol. Chem. 274, 14344-14351) ;
- a mammalian smoothelin (Genbank accession number AH007691) ;
- a mammalian SM20 gene product, especially of human origin (Wax et al., 1996, Lab. Invest. 74, 797-808) ;
- a mammalian Timp4 (Tissue inhibitor of metalloproteinase 4), especially of human origin (Genbank accession number U76456) ;
- a mammalian calponin, with a special preference for the sequence located between positions approximately +138 and approximately +1875 within the first intron of the murine calponin gene (Miano et al., 2000, J. Biol. Chem. 275, 9814-9822).

Preferably, the skeletal alpha-actin gene promoter, and/or the muscle-specific enhancer and/or the gene of interest and/or the vector have the characteristics as defined above. The term "and/or" whereever used herein includes the meaning of "and", "or" and "all or any other combination of the elements connected by said term". The preferred use is intended for the prevention or the treatment of peripheral ischemia.

The invention has been described in an illustrative manner, and it is to be understood that the terminology which has been used is intended to be in the nature of words of description rather than of limitation. Obviously, many modifications and variations of the present invention are possible in light of the above teachings. It is therefore to be understood that within the scope of the appended claims, the invention may be practiced in a different way from what is specifically described herein.

All of the above cited disclosures of patents, publications and database entries are specifically incorporated herein by reference in their entirety to the same extent as if each such individual patent, publication or entry were specifically and individually indicated to be incorporated by reference.

## Figures Legends

Figure 1 is a schematic representation of vectors pTG15331 (1a), pTG15442 (1b) and pTG15680 (1c) which express the luciferase gene under the transcriptional control of the human skeletal alpha-actin promoter in the absence of any enhancer or reinforced by the beta-enolase (ENO) enhancer or the creatine kinase (CK) enhancer.

Figure 2 illustrates promoter activity measurements with the skeletal alpha-actin promoter (pHSA) containing vectors in muscle versus non-muscle cells. The pHSA promoter was used alone (pHSA ; black bars) or in connection with the human creatine kinase enhancer (CK/pHSA; white bars) or the human beta-enolase enhancer (ENO/pHSA; grey bars). HSKMC and C2C12 myoblasts and myotubes, A549 and HUVEC were infected with the different vectors and luciferase activity was determined at day 3. Results are presented as luciferase activity per mg of protein (RLU/mg).

Figure 3 illustrates an *in vivo* evaluation of muscle-specific promoter activity using pHSA-based vectors. Mice were injected with $2x10^8$ iu of the different adenoviruses in tibialis anterior, quadriceps or extensores carpi. Luciferase expression is driven either by the pHSA promoter alone (pHSA ; black bars) or in connection with the human creatine kinase enhancer (CK/pHSA ; white bars) or the human beta-enolase enhancer (ENO/pHSA ; grey bars). Animals were sacrified at day 3 and luciferase activity was determined in the muscles. Results are presented as luciferase activity per mg of protein (RLU/mg).

The following examples serve to illustrate the present invention.

## EXAMPLES

### A. Materials and Methods

The adenoviral genome fragments employed in the different constructs described below are given precisely in accordance with their positions in the nucleotide sequence of the Ad5 genome, as disclosed in Chroboczek et al. (1992, Virol. 186, 280-285).

Standard cloning methods (Sambrook and Russell, 2001, Molecular Cloning : A, Laboratory Manual, Cold Spring Harbor Laboratory Press, Cold Spring Harbor NY) were used to generate the chimeric constructs illustrated hereinafter.

1 Molecular biology

*Polymerase Chain Reaction*

a) PCR amplification of HSA promoter and beta-enolase enhancer sequences.

The PCR kit (Taq Polymerase and buffer) was purchased from Qiagen. Human genomic DNA (1 μg per PCR reaction) was used as template. Specific primers (50 pmol of each per PCR) were used for the HSA promoter (forward primer described in SEQ ID NO : 4 5'-aaacgcgtcgacattggaagtcagcagtcaggc-3' and reverse primer described in SEQ ID NO : 5 5'-ttcgacgtcgacaaagcgcgtgtggctccgga-3') and the beta-enolase enhancer (forward primer described in SEQ ID NO : 6: 5'-aaacgcgtcgacgattcttaggatgggagggtg-3' and reverse primer described in SEQ ID NO: 7: 5'-ttcgacgtcgacgattcttccccactccaagaa-3'). Reaction mix was supplemented with 250 μM (final concentration) dNTPs. The PCR was performed in Perkin Elmer Gene Amp PCR System. The amplification program was composed of an initial denaturation step (5 min, 95°C) followed by 50 cycles of denaturation (1 min, 95°C), primer annealing (1 min, 64°C) and elongation (1 min, 72°C). Finally, an additional step of elongation was performed during 10 min at 72°C.

b) PCR detection of recombinant adenoviral DNA in mice organs.

DNA extracted from mice organs was used as template. Specific primers were designed (forward primer described in SEQ ID NO :8 : 5'-ttccgcgttccgggtcaa-3' and reverse primer described in SEQ ID NO : 9 : 5'-tccagcggttccatcctcta-3'). PCR conditions were almost similar to those described previously, except for annealing temperature (60°C) and number of cycles (30).

c) RT-PCR detection of mouse skeletal α-actin (MSA) and mouse glyceraldehyde-3-phosphate dehydrogenase (MGAPDH) mRNAs in C2C12 myoblasts and myotubes.

The RT-PCR One-Step System kit was purchased from Life Technologies. Total RNA extracts were used as template. Specific MSA primers (forward primer described in SEQ ID NO : 10 : 5'-ccaactgggacgacatggaga-3' and reverse primer described in SEQ ID NO : 11: 5'-atgctgttgtaggtggtctcat-3') and MGAPDH primers (forward primer described in SEQ ID NO : 12 :5'-ccatggagaaggctgggg-3' and reverse primer described in SEQ ID NO: 13 : 5'-caaagttgtcatggatgacc-3') were designed. RT-PCR was composed of a cDNA synthesis step (30 min, 42°C), a pre-denaturation (5 min, 95°C), and 40 cycles of denaturation (15 sec, 95°C), primer annealing (30 sec, 64°C) and elongation (1 min, 72°C).

*DNA cloning*

a) Cloning strategy

The transfer vector contains 5' to 3': a homology sequence with the adenoviral region 1 to 458, the firefly luciferase as reporter gene, the b growth hormone polyadenylation signal (bGHpA) and a homology sequence with the adenoviral region 3511 to 5788. The HSA promoter amplified by PCR carried a *SalI* restriction site at both ends and was cloned in a unique *XhoI* site upstream of the luciferase cDNA. The beta-enolase (ENO) enhancer that was amplified by PCR also carried a *SalI* site at each end, whereas the creatine kinase (CK) enhancer sequence was not amplified by PCR, but subcloned from a pre-existing plasmid (Ribault et al., 2001, Circulation Res. 88, 468-475) by an *XhoI* digestion. Both enhancers were inserted in the *SalI* site upstream of pHSA.

b) Cloning techniques:

DNA fragments were separated on 1% agarose gel and purified with Qiagen Qiaquick Gel extraction Kit.

All restriction enzymes were purchased from New England Biolabs. One unit (U) of enzyme was used to digest 1 μg DNA. Reactions were performed in the appropriate buffer (supplemented with Bovine Serum Albumin (BSA) if necessary) and incubated for 1 h at the recommended temperature.

The 5'-3' polymerase activity of Roche Diagnostics Klenow enzyme was used to generate blunt-ended DNA fragments. Reaction was performed in the appropriate buffer, supplemented with 2 mM dNTPs (final concentration) and 2.5 U of enzyme per μg DNA.

Dephosphorylation was performed to avoid vector self-religation. DNA was incubated for 1 h at 37°C with Roche Diagnostics Calf Intestine Phosphatase (1 U per μg DNA).

Ligation kit (T4 DNA ligase and buffer) was purchased from Gibco BRL. Vector and insert were mixed in a molecular ratio ranking from 1:4 to 1:20, according to molecule size. Reaction was performed in a final volume of 20 µl, and incubated at room temperature during 2 h for cohesive fragment ligation, or overnight for blunt-ended fragment ligation.

*Transformation of* E.coli *DH5α bacterial cells*

Competent cells (100 µl) were mixed with 10 µl of ligation reaction, incubated for 10 min on ice, heat-shocked for 2 min at 37°C, and incubated for 10 additional min on ice. Then 500 µl Luria-Broth medium (LB) were added to the cells, and 100 and 200 µl of this mix were spread on LB agar plates supplemented with 100 µg/ml Ampicillin, as all vectors carry the $Amp^R$ selection gene in their backbone. Plates were incubated overnight at 37°C.

*Plasmid minipreparation*

Plasmid DNA was prepared and purified as described in the Maniatis laboratory manual (1989, Laboratory Manual, Cold Spring Harbor Laboratory Press, Cold Spring Harbor NY).

*Plasmid maxipreparation*

Plasmid maxipreparation was performed with Nucleobond AX cartridges according to manufacturer instructions (Macherey-Nagel, Hoerdt, France). After resuspension in Tris EDTA buffer (TE, 10 mM Tris, 1 mM EDTA, pH 7.5), DNA was quantified with $OD_{260nm}$ measurement (1 OD = 50 µg/ml) and purity was estimated by the $OD_{260nm}$ / $OD_{280nm}$ ratio.

*Homologous recombination in* E.coli *BJ5183*

Viral vectors were constructed as infectious plasmids as described by Chartier et al. (1996, J. Virol. 70, 4805-4810). Briefly, a linearised El-deleted adenoviral genome plasmid was co-transformed with insert (i.e. the expression cassette with homology regions Ad 1-458 and Ad 3511-5788) in 100 µl competent *E.coli* BJ5183 cells in a 1: 10 molecular ratio. Transformation was performed by the heat-shock method.

*Extraction of human genomic DNA from A549 cells*

A549 cells were incubated at 37°C for 2 h in the following buffer: 50 µg/ml Proteinase K, 0.5% SDS, 5 mM EDTA, 5 mM Tris, pH8. After a phenol/dichloromethane extraction, DNA was precipitated with absolute ethanol, and pelleted by centrifugation (16,000 g). Pellet was washed with 70% ethanol, air-dried, resuspended in TE and quantified by $OD_{260nm}$ measurement.

*DNA extraction from mice organs*

After overnight incubation of organs in proteinase K solution (0.2 mg/ml proteinase K, 1% SDS, 5 mM EDTA, 10 mM Tris HCl, 0.3 M sodium acetate, pH8), DNA was prepared as described in the previous paragraph. Appropriate TE volumes were used for resuspension: 300 µl for liver, 100 µl for spleen and lungs, 50 µl for heart DNA respectively.

*RNA extraction from C2C12 myoblasts and myotubes*

RNA was extracted from C2C12 myoblasts and myotubes using the Biogentex RNA Now procedure (Ozyme, Montigny-le Bx, France). Briefly, 1 ml of reagent was added to each well and RNA was solubilized by repetitive pipetting. RNA was then extracted from cell homogenates with chloroform, precipitated with isopropanol, pelleted by centrifugation (10,000 g) and washed with 70% ethanol. RNA was resuspended in ultra-pure water and quantified by $OD_{260nm}$ measurement (1 OD = 40 µg/ml).

<u>2. Cellular biology</u>

Except when notified, all cell lines were cultured in 175 $cm^2$ flasks (Falcon) in Dulbecco's Modified Eagle Medium (DMEM, Life Technologies) supplemented with 10% Fetal Calf Serum (FCS, Life Technologies), 2 mM Glutamine, 40 µg/ml Gentamycine. Cells were incubated at 37°C, 5% $CO_2$. Twice a week, cells were trypsinised after a washing step with Phosphate Buffer saline (PBS, Life Technologies), split 1:2 to 1:10 and replated in new 175 $cm^2$ flasks.

The mouse C2C12 myoblasts (ATCC: CRL-1772) and the human epithelial lung carcinoma A549 cells (ATCC: CCL-185) were purchased from the American Type Culture Collection.

The human retinal PER.C6 complementation cell line (Fallaux et al., 1998, Human Gene Ther. 9, 1909-1917) that expresses the E1 adenoviral region was grown on DMEM supplemented with 10 mM $MgCl_2$ required for cell adhesion to substrate.

The 538 cells (Lusky et al., 1998, J. Virol. 72, 2022-2032) derive from primary human embryonal kidney cells (293, ATCC: CRL-1573). They transcomplement E1 and also express ORF 6 and 7 from adenoviral E4 region.

Human Skeletal Muscle Cells (HSKMC, human myoblasts) and Human Umbilical Vein Endothelial Cells (HUVEC), were purchased from Promocell and cultured in the provided medium.

3. Recombinant adenoviruses production

*PerC6 transfection*

At day 0 (DO), $10^6$ cells (passage inferior to 45) were plated in 6 cm-diameter petri dishes. At D1, the cells were transfected with the $CaPO_4$ precipitation method.

The adenoviral genome produced by homologous recombination in *E.coli* BJ5183 was linearised by a *Swa I* or *Pac I* digestion. After a phenol/dichloromethan extraction, adenoviral DNA was precipitated (with 2 volumes absolute ethanol and 1/10 volume of 3M sodium acetate pH 5), resuspended in sterile TE and quantified. Five µg plasmid were diluted in TE in a final volume of 210 µl, and mixed with 30 µl of sterile-filtered (0.22 µm) 2M $CaCl_2$. This DNA solution was then gently pipeted into 240 µl of HBS 2x (280 mM NaCl, 50 mM HEPES, 1.5 mM $Na_2HPO_4$, pH 7.12). The mix was incubated for 15-30 min at room temperature. The resulting DNA precipitate was then homogeneously distributed on the cells. The plates were incubated overnight. At D2, medium was removed, cells were washed twice with DMEM containing 10% FCS and cultured in DMEM containing 2% FCS until lysis occurred. Transfection was then harvested and frozen.

*Viral pre-stock production*

To amplify the virus, two 75 $cm^2$ flasks were plated with $5x10^6$ PER.C6 cells, and infected in DMEM supplemented with 2% FCS with 500 and 800 µl of transfection, corresponding to a multiplicity of infection (MOI) 1 to 5. The pre-stocks were harvested after lysis and frozen.

*Viral stock production*

Ten 500 $cm^2$ flasks were infected at 50-60 % confluency with 1 ml of pre-stock per flask (MOI 1 to 5) in DMEM containing 2% FCS. After lysis, cells were harvested and centrifuged at 538 g for 10 min. Supernatant was discarded and cells were resuspended in 10 ml medium. In order to enhance virus release, cell membranes were broken by 3 freeze-and-thaw cycles. Cell lysate was then cleared by 2 centrifugations at 4,845 g for 10 min. A cesium chloride (CsCl) gradient was prepared in SW40 polyallomer Beckman Ultracentrifuge tubes: 4 ml of sterile-filtered 1.25 g/ml CsCl were gently pipeted over 4 ml of 1.4 g/ml CsCl. Cleared supernatant was cautiously applied on this gradient and centrifuged for 2 h at 218,000 g and 15°C. The virus was harvested and pipeted on 8 ml of sterile-filtered 1.34 g/ml CsCl. After overnight centrifugation at 218,000 g and 15°C, virus was harvested as before, diluted in 60% sucrose buffer (final sucrose concentration 30%) and injected in a Pierce Slide-A-Lyzer dialysis cassette. The virus was dialysed 3 times for 2 h against 2 litres of the following buffer: 1 M sucrose, 150 mM NaCl, 1 mM MgCl2, 10 mM Tris, Tween 80 1 ml/buffer litre, pH 8.5. The virus stock was finally aliquoted in 1.2 ml Nunc cryotubes, and frozen at -80°C.

*Titration of adenovirus infectious units (iu)*

At D0, 2 Nunc LabTek were plated with $1.5x10^5$ 538 cells per well, in 500 µl DMEM supplemented with 10% FCS, and incubated overnight. At D1, medium was removed and replaced by 200 µl of viral stock serial dilutions ($2x10^{-6}$, $10^{-6}$, $5x10^{-7}$, $2.5x10^{-7}$, $1.25x10^{-7}$, $6.25x10^{-8}$, $3.125x10^{-8}$) in DMEM containing 2% FCS. Two wells were infected per dilution, whereas 2 wells were not infected and used as negative control. After 16 h incubation, medium was removed and cells were fixed in acetone/methanol (v/v) for 10 min at room temperature. Wells were dissociated from slides, which were then immunochemically stained to detect the DNA binding protein (DBP), whose synthesis is induced in infected cells, where viral replication takes place. After 30 min saturation in PBS supplemented with 3% FCS, slides were incubated with the first antibody (α72K B6-8 mouse anti-DBP hybridoma supernatant; Reich et al., 1983, Virology 128, 480-484) for 45 min at room temperature and washed in PBS containing 3% FCS for 10 min. The slides were then incubated for 30 min with a second antibody (rabbit anti-mouse immunoglobulins (Ig) antibody, Dako) and washed as before. Finally a sheep anti-rabbit Ig antibody linked to Fluorescein Isothiocyanate (FITC, Sanofi Pasteur) was used to reveal positive cells (30 min incubation in the darkness). Slides were protected with coverglasses, and conserved at 4°C. Fluorescent cells were counted under UV light and the iu titer was determined by the following formula:

Average number of fluorescent cells per field x 485 x5 / dilution = iu / ml

*Total particles titration*

Virus was diluted in TE supplemented with 0.1% SDS, vortexed for 2 min and centrifuged 5 min at 9,500 g. $OD_{260nm}$ was measured and the total particle titer was given by the formula: $OD_{260nm}$ x dilution x $1.1.10^{12}$ = total particles / ml

The total particles / iu ratio was calculated to evaluate the proportion of empty capsids.

*Viral DNA analysis: Hirth's modified method*

According to viral concentration, 100 to 165 µl of virus were mixed with 2.5 µl Proteinase K (10 mg/ml), 332.5 µl lysis buffer (0.5% SDS, 5 mM EDTA, 5 mM Tris, pH8) and ultra-pure $H_2O$ qsp 500 µl. After overnight incubation at 37°C, proteins were removed by phenol/dichloromethan extraction. Viral DNA was precipitated and resuspended in 60 µl TE. Five restriction enzyme digestions were then performed (10 µl per digestion) to control viral DNA.

*Determination of RCA (Replication Competent Adenoviruses)*

This test was used to ensure that the produced recombinant viruses were defective for replication. At D0, $2x10^6$ A549 cells were plated in 6 cm-diameter petri dishes. At D1, cells were infected with $2x10^6$ iu (MOI =1). When cells were confluent (usually at D3), they were transferred in a 175 $cm^2$ flask and incubated until confluency was reached. If at this time no lysis plaque could be observed, then the viral suspension fullfilled the requirements of the French Commission for Genetically Modified Organisms for non-replicative adenoviral vectors.

<u>4. *In vitro* vector evaluation</u>

*Susceptibility to adenoviral infection*

Cells were plated in 6-well plates, with 3 ml medium per well. For each experiment, 2 to 6 wells were plated per virus (and negative control) and 1 additional well was used to count cells before infection. Cells were infected at MOI ranging from 10 to 1000 with an adenovirus expressing the enhanced Green Fluorescent Protein (eGFP) under the control of the CMV promoter. Virus was diluted in the appropriate medium and added to the corresponding wells. Infection supernatant remained 6 h, and then medium was removed and replaced by fresh medium. Plates were incubated for 3 days at 37°C. At D3, cells were trypsinised, fixed in 3% formaldehyde for 10 min at room temperature, washed twice with PBS and analysed by flow cytometry (Becton Dickinson FACScan).

*Infection conditions*

Infections were performed as described in the previous paragraph, except for reporter expression analysis (luciferase see next paragraph).

HSKMC myoblasts were plated at a density of $6x10^4$ cells per well and infected at MOI 500.

In other experiments, HSKMCs were also fused into myotubes. Cells were plated at a density of $2x10^5$ per well. Growth medium was changed to differentiation medium at confluency; after 6-10 days a syncitium development could be observed. Once differentiated into myotubes, HSKMCs could not be counted: they were nevertheless infected with $3x10^8$ IU (MOI 500 with $6x10^5$ estimated cells).

C2C12 were plated at a density of $2x10^4$ cells per well and infected at MOI 400. After infection, medium was replaced by fresh DMEM supplemented with 10% FCS, in order to avoid differentiation which occurs rapidly in low serum conditions. To evaluate vectors in a myotube context, C2C12 were fused: cells were plated at a density of $10^6$ cells per well, and medium was changed to DMEM containing 2% FCS at confluency. The day after, unlike human myotubes, the differentiated murine cells could be counted. They were then infected at MOI 400.

A549 were plated at a density of $2x10^5$ cells per well and infected at MOI 10. After infection, medium was replaced by fresh DMEM supplemented with 2% FCS.

HUVEC were plated at a density of $2.10^5$ cells per well and infected at MOI 10.

*Luciferase expression measurement*

All vectors carry the firefly luciferase as reporter gene. Three days after infection, medium was removed from the 6-well plates and cells were washed with 1 ml PBS. A volume of 300 µl of Promega Reporter Lysis Buffer (RLB) was added in each well, and plates were frozen for at least 2 h at -80°C to achieve complete cell lysis. Luciferase activity of a 20 µl sample was then quantified using Perkin Elmer opaque multiwell plates, Promega Luciferase Assay Reagent and a MicroLumat EG&G Berthold luminometer. The light produced was measured during 30 sec and averaged to give RLUs (Relative Light Units). Results were normalised according to the total protein content and expressed in RLU/mg. The total protein content of 20 µl cell lysate was quantified using a Bradford test. Samples were mixed with 200 µl of reconstituted BCA Pierce Protein Assay Reagent, and incubated at 37°C for 30 min. A standard curve was established using 0, 5, 10, 15, 20, 25, 30 and 40 µg BSA and $OD_{550nm}$ was read with the SoftMax program.

5. *In vivo* vector evaluation

All animal experiments were performed in a special pathogen-free facility and were conducted according to the French regulations for animal experimentation (Decret No. 87-848, 19.10.1987).

*Promoter strength*

Six-week old female immunocompetent C57BL/6 mice (Iffra-Credo) were injected in *tibialis anterior, quadriceps* or *extensorum carpi et digitorum* muscles with $2x10^8$ iu of each adenovirus in 50 µl of 0.9% NaCl. Mice were sacrificed at D3 after injection, as this time point was found to correspond to maximal reporter activity in a kinetic study (data not shown). Muscles were dissected, frozen in liquid nitrogen and kept at -80°C. Muscles were grinded with a PT3100 Polytron apparatus in 500 µl of RLB. To disrupt cell membranes, 3 freeze-and-thaw cycles were performed. Cell lysates were centrifuged for 15 min at 16,000 g and 4°C. Supernatants were used for luciferase expression measurement and total protein content quantification (as described in section A4) and then frozen at -80°C.

*Promoter specificity*

Mice were injected in the caudal vein with $2x10^9$ iu of adenovirus in 250 µl of 0.9% NaCl. Animals were sacrificed at D3 post injection, and liver, lung, heart and spleen were collected. Half of the organ was frozen in liquid nitrogen and grinded with Polytron in appropriate RLB volume: 1 ml for liver, 500 µl for lung and spleen, 200 µl for heart. Tissue homogenate was then used for luciferase and total protein content quantification. Second half of the organ was incubated overnight in Proteinase K solution and used for DNA extraction (see section A1).

6. Statistical analysis

All results are expressed as mean ± SEM and were analyzed by means of Student's *t*-test. Differences were considered statistically significant at values of $P<0.05$.

**B. RESULTS**

*EXAMPLE 1 : In Vitro* Evaluation of Strength and Specificity of HSA-based Promoters

A series of adenoviral vectors was constructed and produced in PER.C6 cells as described in Materials and Methods. While all expression cassettes contain the luciferase gene as a reporter gene, a non coding exon, an intron and the bGH poly A, they nevertheless differ in enhancer and promoter elements. As illustrated in Figure 1, in pTG 15331, luciferase gene expression is driven by the human skeletal alpha-actin promoter (pHSA) extending from positions -432 to +239 relative to the cap site (+1). Two expression vectors were designed in which the transcriptional activity provided by the HSA promoter is reinforced by a muscle-specific enhancer, i.e. the human beta-enolase (ENO) enhancer extending from positions +504 to +637 relative to the native cap site (pTG15442) or the human creatine kinase (CK) enhancer extending from positions -919 to -711 relative to the native cap site (pTG15680). As a positive control vector, pTG15198 and pTG 15760 contain the luciferase gene driven by the viral IE CMV enhancer/promoter and RSV LTR, respectively. In each case, the luciferase expression cassette was inserted in an E1 and E3 deleted adenoviral backbone in replacement of the adenoviral sequences comprised between positions 459 to 3510. Luciferase gene expression was analysed in both human (HSKMC) and murine (C2C12) myoblasts infected by each type of recombinant adenovirus.

Luciferase expression levels driven by the HSA promoter (no enhancer) reached $5.10^7$ RLU/mg in HSKMC

and 6.4.$10^6$ RLU/mg in C2C12 myoblasts, corresponding to 0.3% and 42.4%, respectively, of the expression levels obtained with the viral RSV promoter (Figure 2). Expression levels increased strongly with differentiation into myotubes, reaching 1.3% and 100% of the RSV-promoted expression in HSKMC and C2C12 myotubes, respectively.

To investigate whether this increase in pHSA-driven expression during differentiation could be correlated with an upregulation of the endogenous mouse skeletal alpha-actin (MSA) transcription, a specific RT-PCR was performed on total RNA extracts of C2C12 in which the difference between myoblasts and myotubes was particularly striking. The MSA-specific signal detected was stronger in differentiated myotubes than in proliferating myoblasts, whereas the GAPDH signal remained unchanged as expected. The increase of luciferase gene expression during differentiation therefore seems to be directly influenced by an endogenous regulation of the promoter.

The addition of the ENO enhancer upstream of pHSA did not lead to any improvement, at least in the adenoviral context, of the luciferase expression in human and murine myoblasts, respectively (Figure 2). Moreover, the addition of the ENO enhancer had no significant effect on pHSA-driven expression in HSKMC myotubes. Nevertheless, a positive effect could be observed in differentiated C2C12 myotubes, with a 2-fold increase of reporter expression. Luciferase activity reached 6.7.$10^8$ RLU/mg, corresponding to 250% of the RSV-driven expression.

The combination of pHSA with the CK enhancer resulted in a statistically significant increase of reporter gene expression in human myoblasts (6.8.$10^7$ RLU/mg vs 5.$10^7$ RLU/mg for pHSA alone). In C2C12 myoblasts, the vector showed the same expression pattern as ENO/pHSA. In human myotubes, expression was slightly increased (1.6.$10^8$ RLU/mg vs 1.2.$10^8$ RLU/mg for pHSA alone). However, in C2C12 myotubes, the presence of the CK enhancer led to a 5-fold increased expression, reaching 570% of the RSV activity.

To demonstrate high muscle specificity of HSA-based promoters being combined with an enhancer as mentioned above, the vectors were tested in A549 and HUVEC cells. Only a background could be detected in both cell types; indeed, the expression driven by pHSA alone in these non-muscle cells was 100 to 1000 times weaker than in differentiated HSKMC or C2C12 myotubes. Moreover, the specificity was further increased 3- to 4-fold by the addition of enhancers.

<u>*EXAMPLE 2 : In Vivo* Evaluation of HSA-based Promoters</u>

*Strength of HSA-based chimeric promoters*

Intramuscular injection of the pHSA-containing vector in mouse *tibialis anterior* (TA), *quadriceps* (Q) and *extensores carpi et digitorum* (EC) led to a similar reporter gene expression in the 3 muscles with 1.3.$10^5$ RLU/mg, corresponding to 5% of the RSV expression (Figure 3). The presence of the ENO enhancer induced a slight increase in promoter activity in TA muscles. On the other hand, the CK/pHSA construct allowed a 2-to 6-fold stronger luciferase expression in all muscles with activities reaching up to 28% of the RSV expression.

*Specificity of HSA-based chimeric promoters*

To demonstrate the strict muscle-specificity of pHSA-driven expression *in vivo,* liver, spleen, heart and lungs of mice were harvested after intramuscular injection of 2x$10^8$ iu of pHSA-based vectors. No reporter gene expression could be observed, although all organs were correctly infected as demonstrated by PCR detection of adenoviral DNA. On the contrary, the CMV-containing vector led to significant reporter expression in all organs, reaching for instance 3.8.$10^7$ RLU/mg in liver. This indicates that unlike the CMV promoter, pHSA-based chimeric regulatory sequences restrict transgene expression to the target tissue after intramuscular injection, although the adenoviral vectors are systemically disseminated.

A massive dose of vector was then injected intravenously in order to evaluate the maximal non-specific expression that could be observed following adenovirus' systemic spreading. With 2x$10^9$ iu, all organs were infected. The adenoviral DNA detection by specific PCR was performed under non-saturating conditions (30 amplification cycles) so that it was possible to illustrate the infection gradient in the organs. Liver was the most infected organ, then spleen and lungs, and finally heart. In liver, the background of expression driven by pHSA alone represented only 0.02 % of the CMV expression. Moreover, pHSA specificity was significantly improved with the use of the enhancers. Indeed, in liver, ENO/pHSA and CK/pHSA led to an expression 8 and 20 times, respectively, weaker than pHSA alone. Similar results were observed in spleen and lungs, whereas in heart, no significant diminution of expression could be observed (the CK/pHSA construct led to a 3-fold higher promoter activity in heart).

These results confirmed the interest of chimeric promoters based on cellular regulatory sequences for the treatment of peripheral ischemia by gene therapy. Indeed, they allow strong and tissue-specific expression in the adenoviral context, thereby insuring efficacy and safety of the gene transfer.

*EXAMPLE 3 : Expression of a therapeutic gene*

The basic plasmid, pTG11236 (Meyer et al., 2000, Gene Ther., 7, 1606-1611) is a pREP4-based plasmid which expresses the luciferase gene under the control of the complete IE1 CMV promoter. The luciferase expression cassette also contains the hybrid 16S/19S SV40 intron (Okayma and Berg, 1983, Mol. Cell. Biol. 3, 280-289) positioned upstream of the luciferase coding region and the SV40 polyA positioned downstream. The pHSA promoter reinforced by the CK enhancer was isolated from pTG15680 (Example 1) as a SacI-BamHI fragment and cloned in replacement of the IE1 CMV promoter. The murine dystrophin gene was isolated from prior art plasmids (Koenig et al., 1987, Cell 50, 509-517 ; Hoffman et al., 1987, Science 238, 347-350 ; Lee et al., 1991, Nature 349, 34-36 ; Bies et al., 1992, Nucleic Acids Res. 20, 1725-1731 ; Clemens et al., 1995, Human Gene Ther. 6, 1477-1485 ; Genbank accession number M68859), and cloned in the resulting plasmid in replacement of the luciferase gene, to give pTG15810. By way of illustration, pTG15810 contains the cer sequence, followed by the kanamycin resistance gene, the Co1E1 replication origin, the CK enhancer fused to the pHSA promoter driving expression of the murine dystrophin gene with a non coding exon and the hybrid 16S/19S SV40 intron positioned upstream of the dystrophin coding region and the SV40 polyA positioned downstream.

Expression of dystrophin produced from the pTG15810 construct was investigated in C2C12 cell line (ATCC CRL 1772). The C2C12 cell line is defective in dystrophin protein. It is derived from C3H mouse myoblasts and differentiates in myotubes after cells reach confluency.

Approximately 24 h before transfection, $5x10^4$ C2C12 cells were plated in 35cm Petri dishes and grown in Dubecco's modified Eagle's medium (DMEM 3g/l glucose) supplemented with 10% fetal calf serum and glutamine 2mM. Medium was changed 3h before transfection and calcium phosphate co-precipitation was performed with 10 or 25μg of plasmid pTG15810 diluted in 100μl of TE-$CaCl_2$ buffer (Tris-HCl 1mM pH 7.5, EDTA 0.05mM, $CaCl_2$ 250mM) and precipited in one volume of HBS 2X buffer (NaCl 280mM, Hepes 50mM, $Na_2HPO_4$ 1.5M). After 16h of incubation, the transfected cells were washed twice with Dulbecco's phosphate buffered saline and the cultures were kept in DMEM medium for 48h or 9 days. Cell cultures transfected with 10μg of plasmid were washed 48h after transfection with Dulbecco's phosphate buffered saline, and then fixed 10 min with methanol/acetone 1:1 at -20°C. Cells transfected with 25μg of plasmid were cultured in DMEM for 9 days to obtain myotube formation, and then fixed with methanol/acetone 1:1 according to the same technical protocol. Petri dishes were stored at -20°C until further use. Detection of dystrophin protein was performed on rehydrated cell cultures by immunofluorescence staining using a dystrophin-specific monoclonal antibody (MANDRA1 ; Sigma) followed by addition of an anti mouse IgG antibody (Dako) and an anti-rabbit IgG antibody labelled with fluoresceine (Sanofi Diagnostic Pasteur).

Fluorescence is detected in myoblast C2C12 cells (48h of culture) as well as in myotubes (9 days of culture), indicating persistence of dystrophin expression throughout the differentiation process when controlled by the CKenhancer and HSA promoter regions.

Dystrophin expression was also assessed by Western blotting following calcium phosphate transfection of pTG15810 in C2C12 cells. For this purpose, $5x10^4$ C2C12 cells were plated in 35cm Petri dishes and grown as described above. Approximately 48 h later, cells were transfected with 5 or 25μg of plasmid pTG15810 using the calcium phosphate co-precipitation technique (see above). After 16h of incubation, the transfected cells were washed twice with DMEM medium and the cultures were kept in this medium for 24h or 8-9 days. Cells were washed twice with PBS and harvested at each time-point by direct lysis with extraction buffer (75 mM Tris, 20% glycerol, 15% SDS, 100 mM DTT, 1x antiprotease Complete (Roche), pH 6.8). Cellular extracts were analysed for the presence of dystrophin by Western blotting using NCL-DYS2 antibody (Novocasta). Dystrophin production appeared weak in myoblast C2C12 cells (24h following transfection) but was clearly detected in myotubes (8-9 days of culture), confirming sustained dystrophin expression when controlled by the CKenhancer and HSA promoter regions.

Plasmid transfer efficiency was also evaluated *in vivo* after intramuscular injection of the dystrophin-expressing plasmid pTG15810 in immunocompetent dystrophin-deficient $mdx^{5cv}$ mice (C57BL/6Ros-$DMD^{mdx\text{-}5cv}$ strain), in conditions where inflammation is maximal, i.e. under notexin pretreatment and repeated injections. Experiments were carried out on 6 to 8-week old animals that have received by intramuscular injection 3 ng of notexin (Sigma), 3 days prior to initial plasmid administration. Eight $mdx^{5cv}$ mice were then injected with 25 μg of pTG15810 plasmid diluted in 25 μl of saline by percutaneous intramuscular single injection in right and left *tibialis.* Half of the treated mice were then reinjected with the same amount of pTG15810 plasmid, three weeks after the first injection. Mice were sacrified either 7 days (once injected mice) or 46 days (twice injected mice) after the first injection. Muscles were collected, embedded in OCT compound (Labonord, Templemars; France), frozen in liquid nitrogen-cooled isopentane and stored at -80°C. Immunohistochemical staining was performed on 5 μm cross-sections collected every 250 μm, to cover the totality of the muscle. All incubations were at room temperature and followed by 3 washes for 5 min in PBS. Sections were fixed in methanol/acetone v/v for 10 min and blocked with 0.5% BSA in PBS for 30 min. They were then incubated with anti-dystrophin monoclonal antibodies (MANDRA1, Sigma) diluted 1:250 for 1.5h, goat anti-mouse immunoglobulin G F $(ab)_2$ fragment $^{biotin}$ diluted 1:500 for 30 min (Immunotech), and streptavidin $^{FITC}$ diluted 1:2000 for 30 min (Caltag Laboratories), all in PBS. Slides were then mounted in the anti-fadding medium Mowiol (Calbiochem/Novabiochem) and observed under an epifluorescnce microscope (Eclipse 800, Nikon). The maximum number of positive fibers per section was considered for each muscle.

As a result, the same number of dystrophin-positive skeletal muscle fibers was seen at 46 days as compared to 7 days in mice injected with pTG15810 plasmid. These results confirm the capability of the transcriptional elements of the present invention to drive expression of dystrophin in muscular dystrophic *mdx*$^{5cv}$ mice.

## Claims

1. A chimeric construct for the expression of a gene of interest in a host cell or organism comprising at least (i) a skeletal alpha-actin gene promoter operably linked with at least (ii) a skeletal muscle-specific enhancer of a human gene.

2. The chimeric construct according to claim 1, wherein said skeletal alpha-actin gene promoter is obtained from a human.

3. The chimeric construct according to claim 2, wherein said human skeletal alpha-actin gene promoter comprises a nucleotide sequence as shown in SEQ ID NO : 1 from positions 1 to 432 or a portion thereof.

4. The chimeric construct according to any of the preceeding claims, wherein said skeletal muscle-specific enhancer is selected from the group consisting of enhancers obtained from the human creatine kinase gene, the human beta-enolase (ENO-3) gene, the human myogenin gene and the human troponin gene.

5. The chimeric construct according to claim 4, wherein said skeletal muscle-specific enhancer is obtained from the human creatine kinase gene and comprises the sequence as shown in SEQ ID NO : 2 or a portion thereof.

6. The chimeric construct according to claim 4, wherein said skeletal muscle-specific enhancer is obtained from the human beta-enolase (ENO-3) gene and comprises the sequence as shown in SEQ ID NO : 3 or a portion thereof.

7. An expression cassette comprising a gene of interest placed under the control of a chimeric construct according to any one of the preceeding claims, allowing its expression in a host cell or organism.

8. The expression cassette according to claim 7, wherein said gene of interest encodes one or more proangiogenic polypeptides, preferably of human origin.

9. The expression cassette according to claim 7 or 8, wherein said gene of interest encodes a ligand capable of activating a ligand-regulated promoter controlling expression of a therapeutic gene.

10. A vector comprising the chimeric construct of any one of claim 1 to 6 or the expression cassette according to any one of claims 7 to 9.

11. The vector according to claim 10, wherein said vector is a viral vector derived from a virus selected from the group consisting of herpes viruses, cytomegaloviruses, foamy viruses, lentiviruses, Semliki forest virus, AAV (adeno-associated virus), poxviruses, retroviruses and adenoviruses.

12. The vector according to claim 11, wherein said viral vector is a replication-defective adenoviral vector.

13. A method for the preparation of viral particles allowing the muscle-specific expression of a gene of interest in a host cell or organism, said method comprising the steps of:

    a) introducing the viral vector of claim 11 or 12 in a permissive cell line;
    b) culturing the permissive cell line obtained in step a) for an appropriate period of time and under suitable conditions to allow the production of viral particles containing said viral vector ;
    c) recovering said viral particles from the cell culture ; and
    d) optionally, purifying the recovered viral particles.

**14.** A viral particle comprising the vector according to claim 11 or 12 or obtainable by the method of claim 13.

**15.** A eukaryotic host cell comprising the chimeric construct of any one of claims 1 to 6, the expression cassette according to any one of claims 7 to 9, the vector according to any one of claims 10 to 12 or infected by the viral particle according to claim 14.

**16.** The eukaryotic host cell according to claim 15, wherein said cell is a skeletal muscle cell.

**17.** A pharmaceutical composition comprising the chimeric construct of any one of claims 1 to 6, the expression cassette according to any one of claims 7 to 9, the vector according to any one of claims 10 to 12, the viral particle according to claim 14 or the eukaryotic host cell according to claim 15 or 16 and, optionally, a pharmaceutically acceptable carrier.

**18.** Use of the chimeric construct of any one of claims 1 to 6, the expression cassette according to any one of claims 7 to 9, the vector according to any one of claims 10 to 12, the viral particle according to claim 14 or the eukaryotic host cell according to claim 15 or 16 for the preparation of a drug for the treatment or the prevention of a disease in a human or animal organism by gene therapy.

**19.** The use according to claim 18 for the preparation of a drug for the treatment or the prevention of a cardiovascular disease and especially peripheral ischemia.

**20.** A method for the treatment of a human or animal organism comprising administering to said organism a therapeutically effective amount of the expression cassette according to any one of claims 7 to 9, the vector according to any one of claims 10 to 12, the viral particle according to claim 14 or the eukaryotic host cell according to claim 15 or 16.

**21.** Use of the chimeric construct of any one of claims 1 to 6, the expression cassette of any one of claims 7 to 9, the vector of any one of claims 10 to 12 or the viral particle according to claim 14 for specific expression of a gene of interest in skeletal muscle cells.

**22.** Use of an expression cassette, a vector or a viral particle comprising a gene of interest placed under the control of at least (i) a skeletal alpha-actin gene promoter and (ii) a muscle-specific enhancer for the preparation of a drug for the treatment or the prevention of a cardiovascular disease in a human or animal organism by gene therapy.

**23.** The use according to claim 22, wherein said skeletal alpha-actin gene promoter has the characteristics defined in claim 2 or 3, said muscle-specific enhancer has the characteristics defined in any one of claims 4 to 6, said gene of interest has the characteristics defined in claim 8 or 9 and/or said vector has the characteristics defined in claim 11 or 12.

**24.** The use according to claim 23, wherein said cardiovascular disease is peripheral ischemia.

Figure 1a

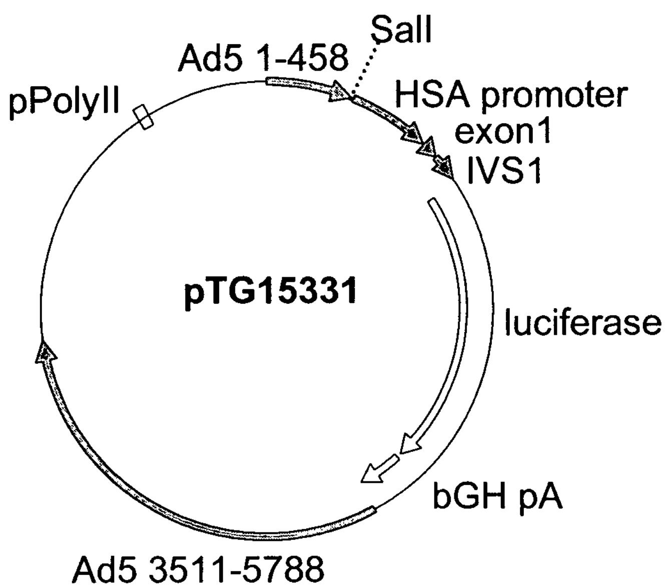
SalI
Ad5 1-458
HSA promoter
exon1
IVS1
pPolyII
pTG15331
luciferase
bGH pA
Ad5 3511-5788

Figure 1b

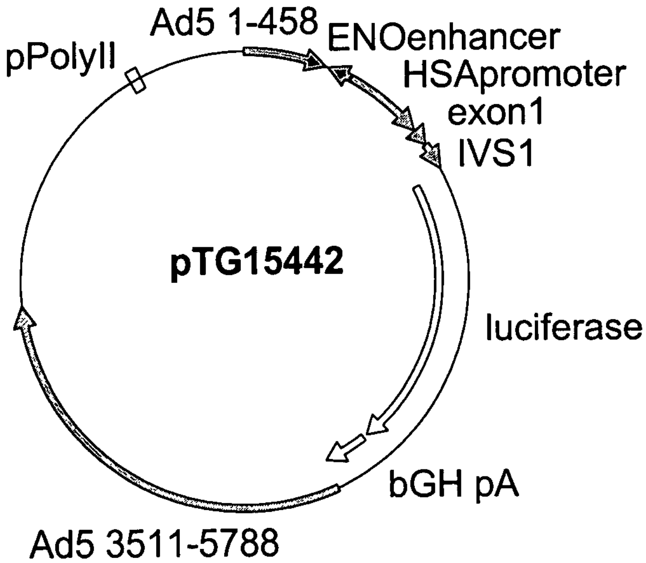


Figure 1c

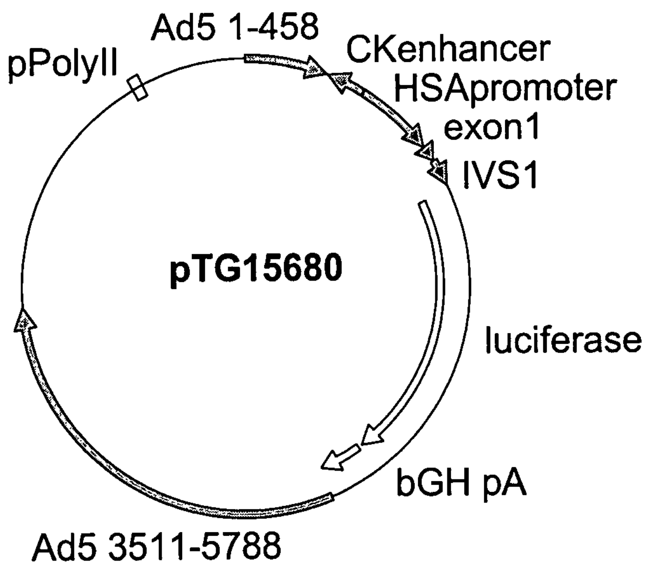

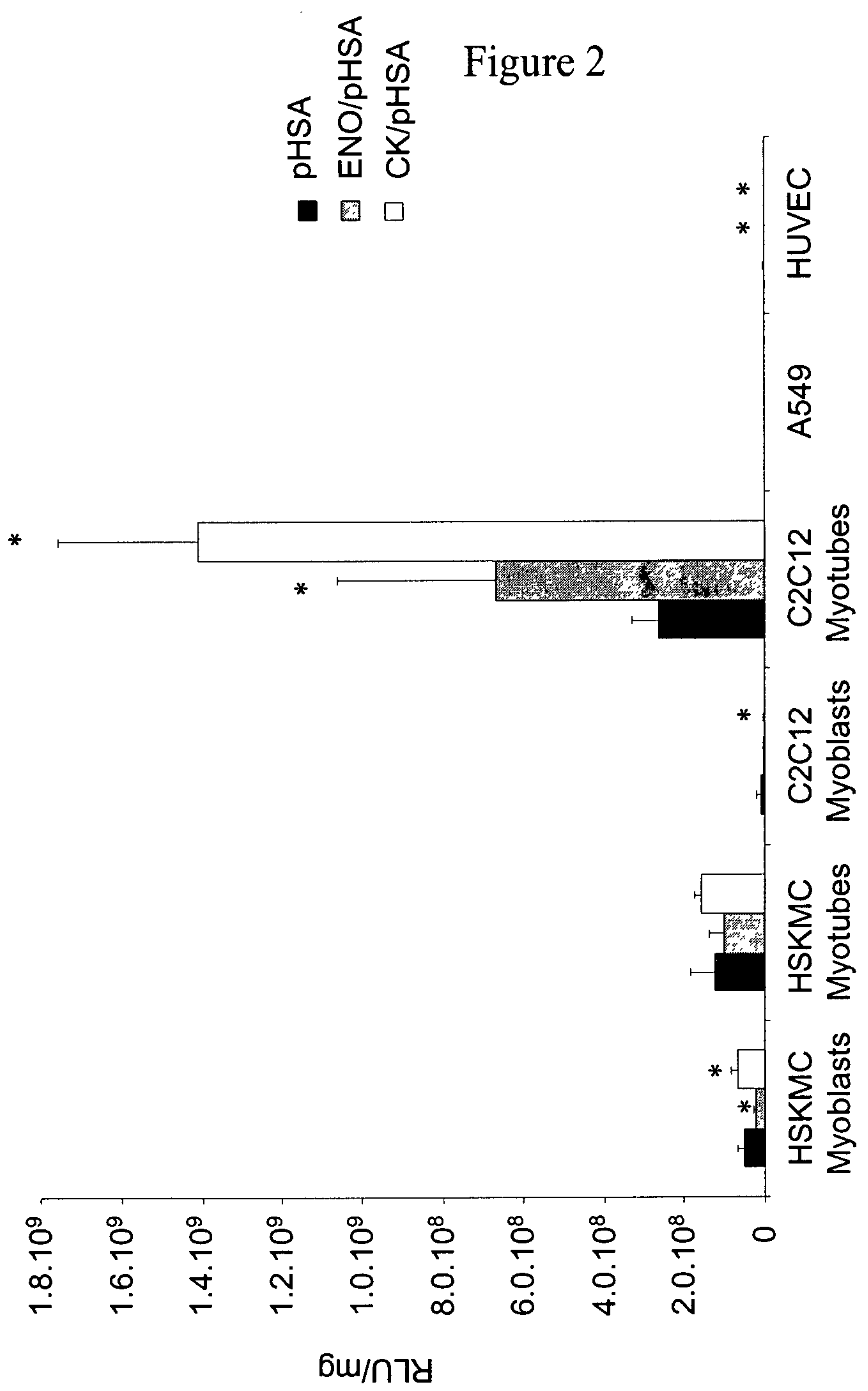
pHSA
ENO/pHSA
CK/pHSA
RLU/mg
1.8.10$^9$
1.6.10$^9$
1.4.10$^9$
1.2.10$^9$
1.0.10$^9$
8.0.10$^8$
6.0.10$^8$
4.0.10$^8$
2.0.10$^8$
0
HSKMC Myoblasts
HSKMC Myotubes
C2C12 Myoblasts
C2C12 Myotubes
A549
HUVEC
*

Figure 2

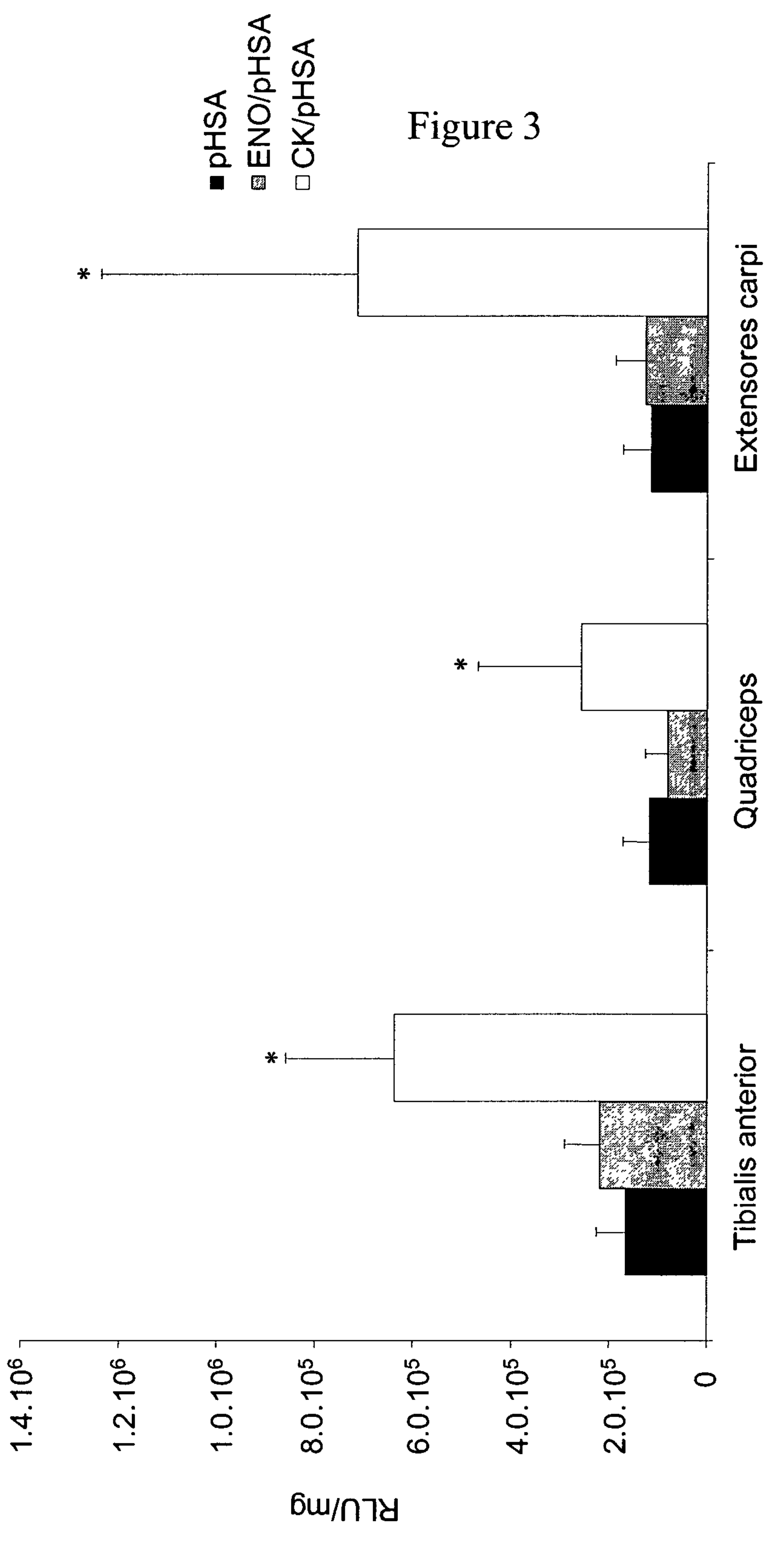

■ pHSA
▨ ENO/pHSA
□ CK/pHSA
RLU/mg
1.4.10^6
1.2.10^6
1.0.10^6
8.0.10^5
6.0.10^5
4.0.10^5
2.0.10^5
0
*
*
*
Tibialis anterior
Quadriceps
Extensores carpi


Figure 3

European Patent Office

**PARTIAL EUROPEAN SEARCH REPORT**

which under Rule 45 of the European Patent Convention shall be considered, for the purposes of subsequent proceedings, as the European search report

Application Number
EP 02 02 2942

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | WANG J M ET AL: "Construction of human factor IX expression vectors in retroviral vector frames optimized for muscle cells." HUMAN GENE THERAPY. UNITED STATES 10 SEP 1996, vol. 7, no. 14, 10 September 1996 (1996-09-10), pages 1743-1756, XP009001678 ISSN: 1043-0342 | 1-5,7, 10,11, 15-18, 20,21 | C12N15/85<br>A61K48/00 |
| Y | * page 1745, left-hand column, paragraph 2 - right-hand column, paragraph 2; figures 1,7; tables 1,2 *<br>* page 1751, left-hand column, line 13 - right-hand column, line 2 *<br>* page 1754, left-hand column, line 41 - line 42 *<br>---<br>-/-- | 12-14, 19,22-24 | |

TECHNICAL FIELDS SEARCHED (Int.Cl.7)

C12N
C07K
A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claims 20 and 21 are directed to a method of treatment of the human/animal body (Article 52(4) EPC), the search has been carried out and based on the alleged effects of the compound/composition.

| Place of search | Date of completion of the search | Examiner |
|---|---|---|
| MUNICH | 3 December 2002 | Rutz, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 02 02 2942

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| Y | MALIK AJAY K ET AL: "Kinetics of recombinant adeno-associated virus-mediated gene transfer." JOURNAL OF VIROLOGY, vol. 74, no. 8, April 2000 (2000-04), pages 3555-3565, XP002223432 ISSN: 0022-538X * page 3555, left-hand column, line 1 - page 3556, right-hand column, paragraph 3; figure 1 * | 12-14 | |
| Y | RIBAULT S ET AL: "CHIMERIC SMOOTH MUSCLE-SPECIFIC ENHANCER/PROMOTERS VALUABLE TOOLS FOR ADENOVIRUS-MEDIATED CARDIOVASCULAR GENE THERAPY" CIRCULATION RESEARCH, GRUNE AND STRATTON, BALTIMORE, US, vol. 88, no. 5, 16 March 2001 (2001-03-16), pages 468-475, XP001057822 ISSN: 0009-7330 * page 473, left-hand column, paragraph 3 - right-hand column, paragraph 1; table 1 * * page 475, left-hand column, paragraph 2 * | 19,22-24 | TECHNICAL FIELDS SEARCHED (Int.Cl.7) |
| X,D | HAGSTROM J NATHAN ET AL: "Improved muscle-derived expression of human coagulation factor IX from a skeletal actin/CMV hybrid enhancer/promoter." BLOOD, vol. 95, no. 8, 15 April 2000 (2000-04-15), pages 2536-2542, XP002223433 ISSN: 0006-4971 * page 2541, left-hand column, line 3 - right-hand column, line 20; table 1 * | 1-3,7, 10-18, 20,21 | |

-/--

EPO FORM 1503 03.82 (P04C10)

European Patent Office

# PARTIAL EUROPEAN SEARCH REPORT

Application Number
EP 02 02 2942

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A,D | FEO S ET AL: "Transcription of the human beta enolase gene (ENO-3) is regulated by an intronic muscle-specific enhancer that binds myocyte-specific enhancer factor 2 proteins and ubiquitous G-rich-box binding factors." MOLECULAR AND CELLULAR BIOLOGY. UNITED STATES NOV 1995, vol. 15, no. 11, November 1995 (1995-11), pages 5991-6002, XP002223434 ISSN: 0270-7306 --- | | |
| A | MUSCAT G E ET AL: "The human skeletal alpha-actin gene is regulated by a muscle-specific enhancer that binds three nuclear factors." GENE EXPRESSION. UNITED STATES 1992, vol. 2, no. 2, 1992, pages 111-126, XP009002150 ISSN: 1052-2166 --- | | TECHNICAL FIELDS SEARCHED (Int.Cl.7) |
| A | WANG JIAN-MIN ET AL: "Persistent systemic production of human factor IX in mice by skeletal myoblast-mediated gene transfer: Feasibility of repeat application to obtain therapeutic levels." BLOOD, vol. 90, no. 3, 1997, pages 1075-1082, XP002223435 ISSN: 0006-4971 --- | | |
| | -/-- | | |

EPO FORM 1503 03.82 (P04C10)

European Patent Office

# PARTIAL EUROPEAN SEARCH REPORT

Application Number
EP 02 02 2942

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A,D | BARNHART K M ET AL: "ENHANCER AND PROMOTER CHIMERAS IN PLASMIDS DESIGNED FOR INTRAMUSCULAR INJECTION: A COMPARATIVE IN VIVO AND IN VITRO STUDY" HUMAN GENE THERAPY, XX, XX, vol. 9, no. 17, 20 November 1998 (1998-11-20), pages 2545-2553, XP001005251 ISSN: 1043-0342 | | |
| A | SHI Q ET AL: "MODULATION OF THE SPECIFICITY AND ACTIVITY OF A CELLULAR PROMOTER IN AN ADENOVIRAL VECTOR" HUMAN GENE THERAPY, XX, XX, vol. 8, 1 March 1997 (1997-03-01), pages 403-410, XP008002788 ISSN: 1043-0342 | | TECHNICAL FIELDS SEARCHED (Int.Cl.7) |
| A,D | WO 00 18908 A (AVENTIS PHARMA SA ;MAHFOUDI ABDERRAHIM (FR); DARTEIL RAPHAEL (FR);) 6 April 2000 (2000-04-06) | | |
| P,A | WO 02 02765 A (RIBAULT SEBASTIEN ;NEUVILLE PASCAL (FR); TRANSGENE SA (FR); MEHTAL) 10 January 2002 (2002-01-10) * page 9, line 1-3 * * page 9, line 15 - page 10, line 6; claim 7 * | | |

EPO FORM 1503 03.82 (P04C10)

## ANNEX TO THE EUROPEAN SEARCH REPORT ON EUROPEAN PATENT APPLICATION NO. EP 02 02 2942

This annex lists the patent family members relating to the patent documents cited in the above-mentioned European search report.
The members are as contained in the European Patent Office EDP file on
The European Patent Office is in no way liable for these particulars which are merely given for the purpose of information.

03-12-2002

| Patent document cited in search report | Publication date | Patent family member(s) | Publication date |
|---|---|---|---|
| WO 0018908 A | 06-04-2000 | FR 2783839 A1 | 31-03-2000 |
| | | AU 5632499 A | 17-04-2000 |
| | | CA 2343922 A1 | 06-04-2000 |
| | | CN 1326506 T | 12-12-2001 |
| | | EP 1115857 A1 | 18-07-2001 |
| | | WO 0018908 A1 | 06-04-2000 |
| | | HU 0105230 A2 | 29-04-2002 |
| | | JP 2002525109 T | 13-08-2002 |
| WO 0202765 A | 10-01-2002 | AU 7061401 A | 14-01-2002 |
| | | WO 0202765 A2 | 10-01-2002 |

EPO FORM P0459

For more details about this annex : see Official Journal of the European Patent Office, No. 12/82

SEQUENCE LISTING

<110> TRANSGENE S.A.

<120> Chimeric promoters for controlling expression in
muscle cells.

<130> promoter HSA and ENO or CK enhancer

<140>
<141>

<160> 13

<170> PatentIn Ver. 2.1

<210> 1
<211> 671
<212> DNA
<213> Homo sapiens

<400> 1
```
attggaagtc agcagtcagg caccttcccg agcgcccagg gcgctcagag tggacatggt 60
tggggaggcc tttgggacag gtgcggttcc cggagcgcag gcgcacacat gcacccaccg 120
gcgaacgcgg tgaccctcgc ccccaccccat cccctccggc gggcaactgg gtcgggtcag 180
gaggggcaaa cccgctaggg agacactcca tatacggccc ggcccgcgtt acctgggacc 240
gggccaaccc gctccttctt tggtcaacgc aggggacccg ggcgggggcc caggccgcga 300
accggccgag ggagggggct ctagtgccca acacccaaat atggctcgag aagggcagcg 360
acattcctgc ggggtggcgc ggagggaatc gcccgcgggc tatataaaac ctgagcagag 420
ggacaagcgg ccaccgcagc ggacagcgcc aagtgaagcc tcgcttcccc tccgcggcga 480
ccagggcccg agccgagagt agcagttgta gctacccgcc caggtagggc aggagttggg 540
aggggacagg gggacagggc actaccgagg ggaacctgaa ggactccggg gcagaaccca 600
gtcggttcac ctggtcagcc ccaggcctcg ccctgagcgc tgtgcctcgt ctccggagcc 660
acacgcgctt t                                                     671
```

<210> 2
<211> 210
<212> DNA
<213> Homo sapiens

<400> 2
```
ggccacccag ggccccgtgg ctgcccttgt aaggaggcga ggcccgagga cacccgagac 60
gcccggttat aattaaccag gacacgtggc gaaccccccct ccaacacctg cccccgaacc 120
cccccatacc cagcgcctcg ggtctcggcc tttgcggcag aggagacagc aaagcgccct 180
ctaaaaataa ctcctttccc ggcgaccgag                                 210
```

<210> 3
<211> 134
<212> DNA
<213> Homo sapiens

<400> 3
```
gattcttagg atgggagggt ggaataagag ctgttctgag tgggggaggg ggctgcgcct 60
gcctctttgg tctgtgacct ttttgtaggg tatttttagc tccagcacct gccttcttgg 120
agtggggaag aatc                                                  134
```

<210> 4
<211> 33

<212> DNA
<213> Artificial Sequence

<220>
<223> Description of Artificial Sequence: forward primer for cloning pHSA promoter

<400> 4
aaacgcgtcg acattggaag tcagcagtca ggc 33

<210> 5
<211> 32
<212> DNA
<213> Artificial Sequence

<220>
<223> Description of Artificial Sequence: reverse primer for cloning pHSA promoter

<400> 5
ttcgacgtcg acaaagcgcg tgtggctccg ga 32

<210> 6
<211> 33
<212> DNA
<213> Artificial Sequence

<220>
<223> Description of Artificial Sequence: forward primer for cloning beta enolase enhancer

<400> 6
aaacgcgtcg acgattctta ggatgggagg gtg 33

<210> 7
<211> 33
<212> DNA
<213> Artificial Sequence

<220>
<223> Description of Artificial Sequence: reverse primer for cloning beta enolase enhancer

<400> 7
ttcgacgtcg acgattcttc cccactccaa gaa 33

<210> 8
<211> 18
<212> DNA
<213> Artificial Sequence

<220>
<223> Description of Artificial Sequence: forward primer for PCr detection of adenoviral DNA

<400> 8
ttccgcgttc cgggtcaa 18

<210> 9
<211> 20
<212> DNA
<213> Artificial Sequence

<220>
<223> Description of Artificial Sequence: reverse primer for PCR detection of recombinant adenoviral DNA

<400> 9
tccagcggtt ccatcctcta 20

<210> 10
<211> 21
<212> DNA
<213> Artificial Sequence

<220>
<223> Description of Artificial Sequence: specificprimer for detection of mouse skeletal alpha actin mRNA

<400> 10
ccaactggga cgacatggag a 21

<210> 11
<211> 22
<212> DNA
<213> Artificial Sequence

<220>
<223> Description of Artificial Sequence: specific prime for detection of r mouse skeletal alpha actin mRNA

<400> 11
atgctgttgt aggtggtctc at 22

<210> 12
<211> 18
<212> DNA
<213> Artificial Sequence

<220>
<223> Description of Artificial Sequence:specific primer for detection of mouse GAPDH mRNA

<400> 12
ccatggagaa ggctgggg 18

<210> 13
<211> 20
<212> DNA
<213> Artificial Sequence

<220>
<223> Description of Artificial Sequence: specific

```
      primer for detection of mouse GAPDH mRNA

<400> 13
caaagttgtc atggatgacc                                                20
```